# EUROPEAN PATENT APPLICATION

(11) **EP 2 954 916 A2**
(43) Date of publication of application: **16.12.2015**
(21) Application number: 15171374.0
(22) Date of filing: 12.08.2011
(51) Int. Cl.: A61M 5/32, A61B 5/151, A61B 5/157

(54) **CLINICAL AND/OR CONSUMER TECHNIQUES AND DEVICES**

(30) Priority: 13.08.2010 US 373764 P
(62) Divisional of application: 11746127.7
(71) Applicant: Seventh Sense Biosystems, Inc., Cambridge, MA 02141 (US)
(72) Inventor: Chickering, Donald, E. lll., Framingham, MA Massachusetts 01701 (US); Levinson, Douglas, A., Sherborn, MA Massachusetts 01770 (US); Davis, Shawn, Santa Monica, California 90404 (US); Haghgooie, Ramin, Arlington, MA Massachusetts 02474 (US); Bernstein, Howard, Cambridge, MA Massachusetts 02138 (US); Walt, David, R., Boston, MA Massachusetts 02116 (US)
(74) Representative: Potter Clarkson LLP

(57) **Abstract**

The present invention generally relates to systems and methods for delivering and/or receiving a substance or substances such as blood, from subjects, e.g., from the skin and/or from other tissues of the body. In some cases, the device may contain a substance transfer component such as needles or microneedles, which can be inserted into the skin or another organ to deliver and/or receiving fluid or other substances from the subject. In some embodiments, the device may include an activator constructed and arranged to insert one or more substance transfer components into the skin or other organ. In certain cases, the device may also include a storage chamber for receiving a fluid received from the subject.

## Description

### RELATED APPLICATIONS

This application claims the benefit of U.S. Provisional Patent Application Serial No. 61/373,764, filed August 13, 2010, entitled "Clinical and/or Consumer Techniques and Devices," by Chickering, *et al.,* incorporated herein by reference.

### FIELD OF INVENTION

The present invention generally relates to systems and methods for delivering and/or receiving a substance or substances such as blood, from subjects, e.g., from the skin and/or from other tissues of the body of a person or animal.

### BACKGROUND

Phlebotomy or venipuncture is the process of obtaining intravenous access for the purpose of intravenous therapy or obtaining a sample of venous blood. This process is typically practiced by medical practitioners, including paramedics, phlebotomists, doctors, nurses, and the like. Substantial equipment is needed to obtain blood from a subject, including the use of evacuated (vacuum) tubes, e.g., such as the Vacutainer^{™} (Becton, Dickinson and company) and Vacuette^{™} (Greiner Bio-One GmBH) systems. Other equipment includes hypodermic needles, syringes, and the like. However, such procedures are complicated and require sophisticated training of practitioners, and often cannot be done in non-medical settings. Accordingly, improvements in methods of obtaining blood or other fluids from the skin are still needed.

### SUMMARY OF THE INVENTION

The present invention generally relates to systems and methods for delivering and/or receiving a substance or substances such as blood, from subjects, e.g., from the skin and/or from other tissues of the body. The subject matter of the present invention involves, in some cases, interrelated products, alternative solutions to a particular problem, and/or a plurality of different uses of one or more systems and/or articles.

In one aspect, the present invention is generally directed to a device for extracting a medium of a subject. In one set of embodiments, the device includes a support structure for application to the skin of the subject, means for extracting a medium of the subject from or through the subject's skin, and an extraction activator associated with the support structure which activates the means for extracting the medium, thereby extracting the medium from or through the subject's skin. In some cases, the extraction activator is constructed and arranged to activate the extracting means not essentially simultaneously with an activation act of the subject, and/or without any activation act of the subject.

In another set of embodiments, the device is a device for delivering and/or withdrawing or receiving fluid from the skin of a subject. The device, according to one set of embodiments, includes a support structure for application to the skin of the subject, transport means associated with the support structure, and an activator constructed and arranged to cause exposure of the transport means to the skin upon activation of the activator. In some cases, the exposure occurs not essentially simultaneously with activation of the activator.

In another set of embodiments, a device is provided for delivering and/or withdrawing or receiving fluid from the skin of a subject. In some embodiments, the device includes a fluid transporter or a substance transfer component, and a timed activator constructed and arranged to insert the fluid transporter or substance transfer component into the skin of the subject.

Yet another set of embodiments includes a device for extracting a medium of a subject. In some cases, the device includes a support structure for application to the skin of the subject, means for extracting a medium of the subject from or through the subject's skin, and an extraction activator associated with the support structure which activates the means for extracting the medium, thereby extracting the medium from or through the subject's skin. In some cases, the extraction activator is constructed and arranged to activate the extracting means not essentially simultaneously with an activation act of the subject, and/or without any activation act of the subject.

The device, in another set of embodiments, is a device for delivering and/or withdrawing or receiving fluid from the skin of a subject. In some embodiments, the device includes a support structure for application to the skin of the subject, transport means associated with the support structure, and an activator constructed and arranged to cause exposure of the transport means to the skin upon activation of the activator. In some cases, the exposure occurs not essentially simultaneously with activation of the activator.

In one set of embodiments, the device is a device for delivering and/or withdrawing or receiving fluid from the skin of a subject. The device may include a fluid transporter or a substance transfer component, a scanner, and an activator constructed and arranged to insert the fluid transporter or substance transfer component into the skin of the subject after receiving a signal from the scanner.

The device, in another set of embodiments, is a device for delivering and/or withdrawing or receiving fluid to or from the skin of a subject. The device includes, in some cases, a fluid transporter or a substance transfer component, a receiver, and an activator constructed and arranged to insert the fluid transporter or substance transfer component into the skin of the subject after receiving a signal from the receiver.

The device, in still another set of embodiments, is able to withdraw or receive a substance from the skin and/or from beneath the skin of a subject. In certain cases, the device includes a fluid transporter or a substance transfer component, a storage chamber for receiving a fluid withdrawn or received from the subject, the storage chamber in fluid communication with the fluid transporter or substance transfer component, and a window in sensing communication with at least a portion of the storage chamber. In some cases, an interrogation signal passing through the window is able to interrogate at least a portion of the storage chamber.

In yet another set of embodiments, the device is a device for withdrawing or receiving a substance from the skin and/or from beneath the skin of a subject. The device in some cases includes a fluid transporter or a substance transfer component, and a test strip in fluid communication with the fluid transporter or substance transfer component.

The device, in accordance with still another set of embodiments, is a device for withdrawing or receiving a substance from the skin and/or from beneath the skin of a subject. The device, in some instances, includes a fluid transporter or a substance transfer component, and a substrate, in fluid communication with the fluid transporter or substance transfer component, having one or more reaction entities thereon able to react with an analyte suspected of being present in fluid withdrawn or received from the subject.

In another set of embodiments, the device is a device for delivering and/or withdrawing or receiving a substance from a subject, comprising a plurality of insertion objects for insertion into the organ of a subject, having a pre-deployed position and a deployed position, and a firing mechanism able to move the insertion objects from the pre-deployed position to the deployed position in a period of time of less than 0.002 seconds, and/or at a velocity of at least 6 meters/second when the plurality of insertion objects first touches the organ during deployment.

In one set of embodiments, the device is a device for delivering and/or withdrawing or receiving a substance from a subject. The device may include, in certain cases, a plurality of insertion objects for insertion into the organ of a subject, having a pre-deployed position and a deployed position, and a reversibly deformable structure, operably linkable to the plurality of insertion objects. In some instances, the reversibly deformable structure is switchable from a first stable configuration, through an unstable configuration, to a second stable configuration. In some embodiments, in the first stable configuration, the insertion objects are not contactable with the organ of the subject, and in the second stable configuration, the insertion objects are insertable in the organ of the subject.

The device, in accordance with another set of embodiments, is a device for delivering and/or withdrawing or receiving a substance from a subject. The device, in some cases, comprises a plurality of insertion objects for insertion into the organ of a subject, having a pre-deployed position and a deployed position, and a reversibly deformable structure, operably linkable to the plurality of insertion objects. In some embodiments, the maximum distance between the reversibly deformable structure and the organ of the subject is no more than 10 mm.

In still another set of embodiments, the device is a device for delivering and/or withdrawing or receiving a substance from a subject. In some instances, the device includes a plurality of insertion objects for insertion into the organ of a subject, having a pre-deployed position and a deployed position, and a reversibly deformable structure, operably linkable to the plurality of insertion objects. In some cases, the device may comprise at least one of: a largest lateral dimension, when the device is positioned for extraction of the medium, parallel to the extraction area, of no more than about 6 cm; or a largest vertical dimension, extending from the organ of the subject when the device is positioned for extraction of the medium, of no more than about 1.5 cm; or a mass of no more than about 25 g, absent the medium.

The device, in yet another set of embodiments, is a device for delivering and/or withdrawing or receiving a substance from a subject. The device, in some instances, includes a plurality of insertion objects for insertion into the organ of a subject, having a pre-deployed position and a deployed position, and a triggering mechanism able to move the insertion objects from the pre-deployed position to the deployed position and to accelerate the insertion objects, during at least one period of time during movement from the pre-deployed position toward the deployed position, at a rate of at least 100,000 meters/second².

In still another set of embodiments, the device is a device for delivering and/or withdrawing or receiving a substance from a subject. The device, in some instances, includes a plurality of insertion objects for insertion into the organ of a subject, having a pre-deployed position and a deployed position, and a triggering mechanism able to move the insertion objects from a fully pre-deployed position to a fully deployed position, wherein the distance between the fully pre-deployed position to the fully deployed position is no more than 5,000 microns.

The device, in yet another set of embodiments, is a device able to deliver and/or withdraw or receive a substance from a subject. The device may include a plurality of insertion objects for insertion into the organ of a subject, having a pre-deployed position and a deployed position, substantially each of the insertion objects protruding from a base and defining a length from the base, and the plurality of insertion objects defining an average length of no more than 1,000 microns, and a triggering mechanism able to move the insertion objects a fully pre-deployed position to a fully deployed position with a force sufficient to insert the plurality of insertion objects into or through the organ to an average depth of at least 60% the average length of the plurality of insertion objects.

According to still another set of embodiments, the device is a device for delivering and/or withdrawing or receiving a substance from a subject, including a plurality of insertion objects for insertion into the organ of a subject, having a pre-deployed position and a deployed position, and a reversibly deformable structure, operably linkable to the plurality of insertion objects, the reversibly deformable structure switchable from a stored energy position, through an actuation energy barrier at an energy higher than the stored energy position, to a deployed energy position at an energy level lower than the actuation energy barrier, wherein the stored energy position is associated with the pre-deployed position of the insertion objects, and the deployed energy position is associated with the deployed position of the insertion objects.

The device, in one set of embodiments, is a device for analysis of an extractable medium of a subject. In some embodiments, the device includes a support structure for application to the skin of the subject, the support structure comprising means for extracting a medium of the subject from or through the subject's skin, and a signal structure for generating a signal indicative of the determination of at least one analyte of the medium. In certain cases, the support structure and the signal structure are constructed and arranged to be connectable and/or detachable from each other readily by the subject.

In another set of embodiments, the device is a device for delivering and/or withdrawing or receiving fluid from the skin of a subject. In some cases, the device includes a support structure for application to the skin of the subject. The support structure may comprise transport means for delivery and/or withdrawal or receiving of fluid from the skin of the subject, and a signal structure for generating a signal indicative of fluid delivered to and/or withdrawn or received from the skin of the subject. In some instances, the support structure and the signal structure are constructed and arranged to be connectable and/or detachable from each other.

The device, in yet another set of embodiments, is a device for withdrawing or receiving a substance from the skin and/or from beneath the skin of a subject. In some instances, the device includes a first portion having a fluid transporter or substance transfer component, and a storage chamber for receiving a fluid withdrawn or received from the subject via the fluid transporter or substance transfer component, and a second portion able to create a pressure differential. In some cases, the first and second portions can be separated from each other without breakage or tools.

The device, in still another set of embodiments, is a device for delivering and/or withdrawing or receiving fluid from the skin of a subject. The device, in some embodiments, includes a plurality of microneedles mounted on a rotatable structure, and an activator constructed and arranged to insert one or more of the microneedles into the skin of the subject.

In yet another set of embodiments, the device is a device for extracting blood of a subject. In some embodiments, the device includes a support structure for application to the skin of the subject, means for extracting blood of the subject from or through the subject's skin, and means for altering, from the skin of the subject, residual blood and/or the appearance of residual blood such that when the support structure is removed from the skin of the subject visible residual blood is not present in an amount that is visible to the unassisted eye of the subject. In some cases, the support structure includes at least a cover between the subject's line of vision and any blood extracted by the device. In certain embodiments, application, use, and removal of the device from the skin of the subject produces no blood visible by the unassisted eye of the subject.

In another set of embodiments, the device is a device for delivering and/or withdrawing or receiving fluid from the skin of a subject, including a support structure for application to the skin of the subject, transport means associated with the support structure, and altering means for altering the appearance of blood on the skin of the subject after removal of the device from the subject.

The device, according to one set of embodiments, is a device for delivering and/or withdrawing or receiving fluid from the skin of a subject. The device, in some embodiments, may include transport means for delivery and/or withdrawal or reception of fluid from the skin of a subject, and altering means for altering the appearance of blood on the subject after removal of the device from the subject.

In accordance with another set of embodiments, the device is a device for delivering and/or withdrawing or receiving fluid from the skin of a subject, including a support structure for application to the skin of the subject, transport means associated with the support structure, and a chemical for altering the appearance of blood on the subject.

In one set of embodiments, the device is able to withdraw or receive a substance from the skin and/or from beneath the skin of a subject. In some cases, the device may include a plurality of microneedles, and a shield able to cover a portion of the microneedles when the microneedles are not being used to deliver and/or withdraw or receive fluid to or from the skin of a subject.

The device, in another set of embodiments, is a device for withdrawing or receiving a substance from the skin and/or from beneath the skin of a subject. The device, in some cases, may include one or more microneedles, and a sleeve into which the microneedles can be withdrawn or received into when the microneedles are not being used to deliver and/or withdraw or receive fluid from the skin of a subject.

Still another set of embodiments is generally directed to a device for withdrawing or receiving fluid from the skin of a subject. The device, in certain cases, includes a support structure for application to the skin of the subject, transport means associated with the support structure, and a stabilizing agent for stabilizing the fluid withdrawn or received from the skin.

Yet another set of embodiments is generally directed to a device for withdrawing or receiving fluid from the skin of a subject. In certain embodiments, the device includes a support structure for application to the skin of the subject, transport means associated with the support structure, and an anticoagulant for stabilizing the fluid withdrawn or received from the skin.

In one set of embodiments, the device is a device for withdrawing or receiving a substance from the skin and/or from beneath the skin of a subject. The device may include a fluid transporter or a substance transfer component, and a storage chamber for receiving a fluid withdrawn or received from the subject, the storage chamber in fluid communication with the fluid transporter or substance transfer component. In some cases, the storage chamber contains a stabilizing agent for stabilizing the fluid withdrawn or received from the skin.

The device, in another set of embodiments, is generally directed to a device for withdrawing or receiving a substance from the skin and/or from beneath the skin of a subject. In some embodiments, the device includes a fluid transporter or a substance transfer component, a storage chamber for receiving a fluid withdrawn or received from the subject, and a reservoir able to contain a fluid deliverable to the storage chamber. In certain instances, the storage chamber is in fluid communication with the fluid transporter or substance transfer component.

In accordance with still another set of embodiments, the device is a device for withdrawing or receiving a substance from the skin and/or from beneath the skin of a subject. In certain instances, the device includes a fluid transporter or a substance transfer component, and a storage chamber for receiving a fluid withdrawn or received from the subject. In some cases, the storage chamber is in fluid communication with the fluid transporter or substance transfer component. The storage chamber may also contain a fluid, at least in certain embodiments.

According to yet another set of embodiments, the device is a device for extraction of a medium from a subject. In some cases, the device includes a support structure for application to the skin of the subject, and means for extracting a medium of the subject from or through the subject's skin at an extraction area. In some embodiments, the device comprises at least one of a largest lateral dimension, parallel to the extraction area, of no more than about 6 cm, or a largest vertical dimension, extending from the skin of the subject when the device is positioned for extraction of the medium, of no more than about 1.5 cm, or a mass of no more than about 25 g.

In still another set of embodiments, the device is a self-contained device for delivering and/or withdrawing or receiving fluid from the skin of a subject. In certain embodiments, the device includes a support structure for application to the skin of the subject. The support structure may comprise, in certain cases, transport means for delivery and/or withdrawal or received of fluid from the skin of a subject. In some instances, the self-contained device, when affixed to the skin, may also comprise at least one of a largest lateral dimension of no more than about 6 cm, a largest vertical dimension, extending from the skin of the subject when the device is applied to the subject, of no more than about 1.5 cm, or a mass of no more than about 25 g.

The invention is generally directed to various methods, according to another aspect. In one set of embodiments, the method includes acts of applying a device to the skin of a subject able to deliver and/or withdraw or receive fluid from the subject, and activating the device. In some embodiments, after a period of time after activation, the device exposes a transport means for delivering and/or withdrawing or receiving fluid to the skin of the subject

The method, in another set of embodiments, includes acts of applying a device to the skin of a subject able to deliver and/or withdraw or receive fluid to and/or from the skin of the subject, and activating the device. In some cases, after a period of time after activation, the device inserts a fluid transporter or a substance transfer component for delivering and/or withdrawing or receiving fluid into the skin of the subject.

In yet another set of embodiments, the method includes acts of applying a device to the skin of a subject able to deliver and/or withdraw or receive fluid from the subject, and activating the device. In some embodiments, after a period of time after activation, the device inserts one or more microneedles into the skin of the subject

The method, in still another set of embodiments, includes acts of applying a device to the skin of a subject able to deliver and/or withdraw or receive fluid from the subject, and activating the device. The device may be able to automatically insert a fluid transporter or a substance transfer component into the skin of the subject at a specific time of day without any external intervention, at least in some cases.

In yet another set of embodiments, the method includes acts of applying a device to the skin of a subject able to deliver and/or withdraw or receive fluid from the subject, and activating the device. In certain embodiments, the device automatically inserts a fluid transporter or a substance transfer component into the skin of the subject in response to an action taken by the subject, in some cases without any external intervention.

The method, in accordance with another set of embodiments, includes acts of applying a device to the skin of a subject able to deliver and/or withdraw or receive fluid from the subject, and activating the device. In some embodiments, after a period of time after activation, the device is able to expose a transport means for delivering and/or withdrawing or receiving fluid to the skin of the subject.

The method, in yet another set of embodiments, includes acts of providing a device to the skin of a subject able to deliver and/or withdraw or receive fluid to or from the subject, exposing the device to a scannable target, and applying the device to the skin of a subject.

In one set of embodiments, the method includes acts of applying a device to the skin of a subject able to deliver and/or withdraw or receive fluid from the subject, delivering and/or withdrawing or receiving fluid from the subject using the device; and removing only one of the support structure and the signal structure from the skin of the subject. In some embodiments, the device includes a support structure comprising transport means for delivery and/or withdrawal or receiving of fluid from the skin of a subject, and a signal structure for generating a signal indicative of fluid delivered to and/or withdrawn or received from the skin of the subject.

The method, in yet another set of embodiments, includes acts of applying a device to the skin of a subject able to deliver and/or withdraw or receive fluid from the subject, delivering and/or withdrawing or receiving fluid from the subject using the device, and removing only one of the first portion or the second portion from the device while the device is on the skin of the subject. In some cases, the device may include a first portion having a fluid transporter or a substance transfer component, and a storage chamber for receiving a fluid withdrawn or received from the subject via the fluid transporter or substance transfer component, and a second portion able to create a pressure differential.

According to still another set of embodiments, the method includes acts of applying a device to the skin of a subject able to deliver and/or withdraw or receive fluid from the subject, delivering and/or withdrawing or receiving fluid from the skin of the subject using the device, and altering the appearance of blood on the skin of the subject using the device.

In yet another set of embodiments, the method includes acts of applying a self-contained device to the skin of a subject able to deliver and/or withdraw or receive fluid from the subject, and activating the device. In some cases, the self-contained device, when affixed to the skin, comprises at least one of (1) a largest lateral dimension of no more than about 6 cm, (2) a largest vertical dimension, extending from the skin of the subject when the device is applied to the subject, of no more than about 1.5 cm, or (3) a mass of no more than about 25 g.

In another aspect, the present invention encompasses methods of making one or more of the embodiments described herein, for example, devices for delivering and/or withdrawing or receiving blood or other substances to or from a subject. In still another aspect, the present invention encompasses methods of using one or more of the embodiments described herein, for example, devices for delivering and/or withdrawing or receiving blood or other substances to or from a subject.

Other advantages and novel features of the present invention will become apparent from the following detailed description of various non-limiting embodiments of the invention when considered in conjunction with the accompanying figures. In cases where the present specification and a document incorporated by reference include conflicting and/or inconsistent disclosure, the present specification shall control. If two or more documents incorporated by reference include conflicting and/or inconsistent disclosure with respect to each other, then the document having the later effective date shall control.

### BRIEF DESCRIPTION OF THE DRAWINGS

Non-limiting embodiments of the present invention will be described by way of example with reference to the accompanying figures, which are schematic and are not intended to be drawn to scale. In the figures, each identical or nearly identical component illustrated is typically represented by a single numeral. For purposes of clarity, not every component is labeled in every figure, nor is every component of each embodiment of the invention shown where illustration is not necessary to allow those of ordinary skill in the art to understand the invention. In the figures:
Figs. 1A-1B illustrate devices according to certain embodiments of the invention;
Figs. 2A-2C illustrate devices according to various embodiments of the invention; and
Fig. 3 illustrates yet another embodiment of the invention in which a device is actuated by a reversibly deformable structure.

### DETAILED DESCRIPTION

Systems and methods are shown for delivering to and/or receiving a substance or substances such as blood, from subjects, e.g., from the skin and/or to or from other tissues of the body. In some cases, the device may contain a fluid transporter or a substance transfer component such as needles or microneedles, which can be inserted into the skin or another organ to deliver and/or receive fluid or other substances to or from the subject. In some embodiments, the device may include an activator constructed and arranged to insert one or more fluid transporters or substance transfer components into the skin or other organ. In certain cases, the device may also include a storage chamber for receiving a fluid received from the subject.

In certain aspects, the present invention generally relates to devices for receiving a substance from a subject, e.g. received from the skin and/or from beneath the skin of the subject, and/or for delivering a substance to a subject, e.g. delivering a substance to the skin and/or to a location beneath the skin of a subject. In some cases, a substance may be delivered to and/or received from an organ. Details of such devices follow. The device, in some cases, may be interfaced with external equipment to determine an analyte contained within a fluid contained within or collected by the device. For example, the device may be mounted on an external holder, the device may include a port for transporting fluid out of the device, the device may include a window or integral mechanism for interrogating or facilitating interrogation or sampling of a fluid contained within the device, or the like.

The received fluid may be any suitable bodily fluid, such as interstitial fluid, other skin-associated material, mucosal material or fluid, whole blood, perspiration, saliva, plasma, tears, lymph, urine, plasma, or any other bodily fluid, or combinations thereof. Substances received from a subject can include solid or semi-solid material such as skin, cells, or any other substance from the subject. Substances that can be delivered to a subject in accordance with some embodiments of the invention include diagnostic substances, therapeutic substances such as drugs, and the like. Various embodiments of the invention are described below in the context of delivering or receiving a fluid, such as blood, from or through the skin. It is to be understood that in all embodiments herein, regardless of the specific exemplary language used (e.g., receiving blood), the devices and methods of other embodiments of the invention can be used for receiving any substance from the skin and/or from beneath the skin of the subject, and/or for delivering any substance to the subject, e.g. to the skin and/or a location beneath the skin of the subject. For example, in some cases, a substance may be delivered to and/or received from an organ. It should also be understood that, in some cases, fluid may be present beneath the skin, e.g., in the fatty or muscle layers below the skin. Accordingly, descriptions herein of delivering and/or receiving fluid "in the skin" should also be understood to include, in other embodiments, the deliver and/or receiving of fluid into layers directly beneath the skin, or in other organs within the body.

In one aspect, the present invention is generally directed to devices and methods for receiving or extracting blood or other bodily fluids from a subject, e.g., from the skin and/or from beneath the skin, using devices having a substance transfer component (which may include, for example, one or more microneedles and/or other skin insertion objects). The device may also contain, in some embodiments, a storage chamber having an internal pressure less than atmospheric pressure prior to receiving blood or other bodily fluids. In some cases, the device may pierce the skin of the subject, and fluid can then be delivered and/or received from the subject. The subject is usually human, although non-human subjects may be used in certain instances, for instance, other mammals such as a dog, a cat, a horse, a rabbit, a cow, a pig, a sheep, a goat, a rat (e.g., *Rattus Norvegicus*), a mouse (e.g., *Mus musculus*), a guinea pig, a hamster, a primate (e.g., a monkey, a chimpanzee, a baboon, an ape, a gorilla, etc.), or the like.

In some cases, the device can be applied to the skin, and activated to receive fluid from the subject. The device, or a portion thereof, may then be processed to determine the fluid and/or an analyte within the fluid, alone or with an external apparatus. For example, fluid may be received from the device, and/or the device may contain sensors or agents able to determine the fluid and/or an analyte suspected of being contained in the fluid.

Some embodiments encompass the determination of a condition of a subject. Bodily fluids and/or other substances may be analyzed, for instance, as an indication of a past, present and/or future condition of the subject, or to determine conditions that are external to the subject. Determination may occur, for instance, visually, tactilely, by odor, via instrumentation, etc. Some embodiments are thus generally directed to various devices for delivering and/or receiving blood, or other bodily fluids or substances from a subject, e.g., from the skin of a subject, from beneath the subject, etc. Accordingly, in the description that follows, the discussion of blood is by way of example only, and in appropriate embodiments, other fluids or substances may be delivered to and/or received from the skin, or other organs, in addition to and/or instead of blood.

In one set of embodiments, the device includes a substance transfer component able to deliver to or receive fluid from the subject. As used herein, "substance transfer component" is any component or combination of components that facilitates movement of a substance or a fluid from one portion of the device to another, and/or from the device to the subject or vice versa. The substance transfer component may include an opening of any size and/or geometry that is constructed to receive fluid into the device. For example, an opening of a substance transfer component may lie in a two-dimensional plane or the opening may include a three-dimensional cavity, hole, groove, slit, etc. In some embodiments, the substance transfer component may also include one or more microneedles or other skin insertion objects, arranged to cause fluid to be released from the subject, e.g., by piercing the skin of a subject. In some embodiments, if fluid may partially or fully fill an enclosure surrounding a skin insertion object or other object, then the enclosure can define at least part of a substance transfer component. A substance transfer component may include any other suitable fluid transporter or flow activator. Other components including partially or fully enclosed channels, microfluidic channels, tubes, wicking members, vacuum containers, etc. can be, or be a part of, a substance transfer component.

The fluid may be received from and/or through the skin of a subject (or other organ or mucosal surface, etc.). The substance transfer component may be, for example, one or more needles and/or microneedles, a hygroscopic agent, a cutter or other piercing element, an electrically-assisted system, or the like, e.g., as discussed in detail herein. If needles or microneedles are used, they may be solid or hollow, i.e., blood or other fluid may travel in and/or around the needles or microneedles into or from the device. In some cases, the needles or microneedles may also be removed from the subject, e.g., after insertion into the skin, for example, to increase the flow of blood or other fluids from the subject. In one set of embodiments, the substance transfer component includes solid needles that are removed from the skin and a cup or channel to direct the flow of blood or other bodily fluids.

In some aspects, the device may include a support structure, such as a housing. The housing may be used, as discussed herein, for applying the substance transfer component to the surface of the skin of the subject, e.g., so that fluid may be delivered and/or received from the skin of the subject. In some cases, the housing may immobilize the substance transfer component such that the substance transfer component cannot move relative to the housing; in other cases, however, the substance transfer component, or a portion thereof, may be able to move relative to the housing. In one embodiment, as a non-limiting example, the substance transfer component is immobilized relative to the housing, and the deployment actuator is positioned within the device such that application of the device to the skin causes at least a portion of the substance transfer component to pierce the skin of the subject. In some cases, the housing encloses a deployment actuator.

In some embodiments, the deployment actuator, or a portion of the deployment actuator, may move from a first position to a second position. For example, the first position may be one where the deployment actuator has attached thereto a substance transfer component that is not in contact with the skin (e.g., a skin insertion object of the substance transfer component may be contained within a recess of the substance transfer component), while the second position of the deployment actuator may be one where the substance transfer component does contact the skin, e.g., to pierce the skin. The deployment actuator may be moved using any suitable technique, e.g., manually, mechanically, electromagnetically, using a servo mechanism, or the like. In one set of embodiments, for example, the deployment actuator may be moved from a first position to a second position by pushing a button on the device, which causes the deployment actuator to move (either directly, or through a mechanism linking the button with the deployment actuator). Other mechanisms (e.g., dials, levers, sliders, etc., as discussed herein) may be used in conjunction of or instead of a button. In another set of embodiments, the deployment actuator may be moved from a first position to a second position automatically, for example, upon activation by a computer, upon remote activation, after a period of time has elapsed, or the like. For example, in one embodiment, a servo connected to the deployment actuator is activated electronically, moving the deployment actuator from the first position to the second position. In some cases, the deployment actuator may include a triggering mechanism that initiates deployment.

In some cases, the deployment actuator and/or the substance transfer component may also be moved from the second position to the first position (or some other position). For example, after fluid has been delivered and/or received from the skin, e.g., using a substance transfer component, the deployment actuator may be moved, which may move the substance transfer component away from contact with the skin. The deployment actuator may be moved from the second position to the first position using any suitable technique, including those described above, and the technique for moving the deployment actuator from the second position to the first position may be the same or different as that moving the deployment actuator from the first position to the second position.

In some cases, the device may be able to draw skin towards the substance transfer component. For example, in one set of embodiments, the device may include a vacuum interface or region. The interface or region may be connected with a vacuum source (external and/or internal to the device), and when a vacuum is applied, skin may be drawn towards the device, e.g., for contact with a substance transfer component, such as one or more needles or microneedles.

In one set of embodiments, the device includes a deployment actuator able to drive a substance transfer component into the skin, e.g., so that the device can receive a fluid from the skin of a subject, and/or so that the substance transfer component can deliver a substance to a subject, e.g. deliver a substance to the skin and/or to a location beneath the skin of a subject. The deployment actuator may be a structure that can be deformed using unaided force (e.g., by a human pushing the structure), or other forces (e.g., electrically-applied forces, mechanical interactions or the like), but is able to restore its original shape after the force is removed or at least partially reduced. For example, the structure may restore its original shape spontaneously, or some action (e.g., heating) may be needed to restore the structure to its original shape. In one set of embodiments, the deployment actuator may include a flexible concave member or a reversibly deformable structure that is moveable between a first configuration and a second configuration. The deployment actuator may be formed out a suitable elastic material, in some cases. For instance, the structure may be formed from a plastic, a polymer, a metal, etc. In one set of embodiments, the structure may have a concave or convex shape. For instance, the edges of the structure may be put under compressive stress such that the structure "bows" out to form a concave or convex shape. A person pushing against the concave or convex shape may deform the structure, but after the person stops pushing on the structure, the structure may be able to return to its original concave or convex shape, e.g., spontaneously or with the aid of other forces as previously discussed. In some cases, the device may be bistable, i.e., having two different positions in which the device is stable.

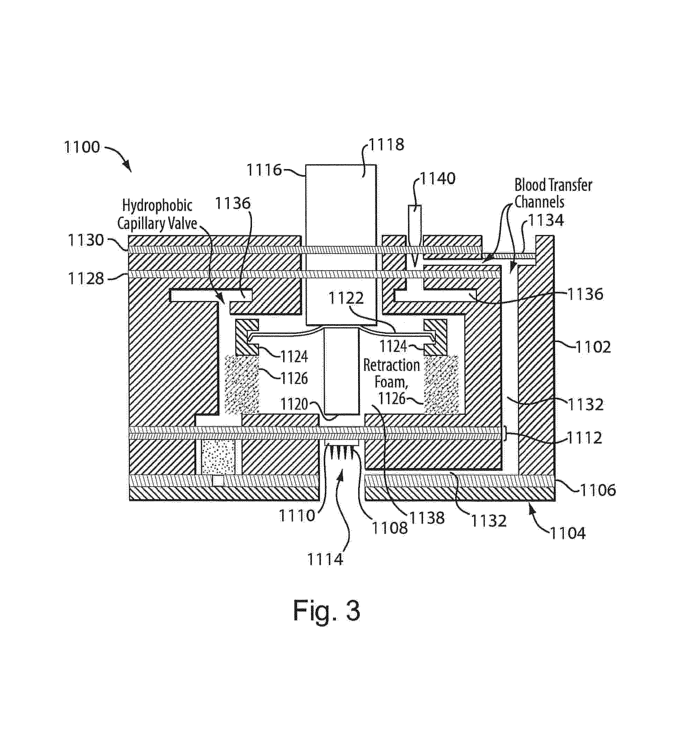

As another example, referring now to Fig. 3, a device 1100 is illustrated schematically in which a substance transfer component is driven by a deployment actuator. In Fig. 3, device 1100 includes a housing 1102 defining a plurality of chambers and channels. In other embodiments (not shown) a plurality of components that can be separable from and attachable to each other (e.g., modular components) can together define the device and together define a series of channels and compartments necessary for device function. See, e.g., U.S. Patent Application Serial No. 12/716,233, filed March 2, 2010, entitled "Systems and Methods for Creating and Using Suction Blisters or Other Pooled Regions of Fluid within the Skin," by Levinson, *et al.*; U.S. Patent Application Serial No. 12/716,226, filed March 2, 2010, entitled "Techniques and Devices Associated with Blood Sampling," by Levinson, *et al.*; or U.S. Patent Application Serial No. 12/716,229, filed March 2, 2010, entitled "Devices and Techniques Associated with Diagnostics, Therapies, and Other Applications, Including Skin-Associated Applications," by Bernstein, *et al.,* each incorporated herein by reference.

In the specific device illustrated, device 1100 includes a surface 1104 for positioning the device proximate the skin of a subject during use. Where desired in certain embodiments, the device can include an adhesive layer 1106 where the adhesive is selected to be suitable for retaining the device in a relatively fixed position relative to the skin during use, but may allow for relatively easy removal of the device from the skin following use. Specific non-limiting examples of adhesives are discussed below. The adhesive also can be selected to assist in maintaining a vacuum within portions of the device proximate the skin as will be understood.

In Fig. 3, device 1100 includes a substance transfer component 1108. The substance transfer component may be or include, for example, a skin insertion object or other suitable object as discussed herein. Specific non-limiting examples include needles or microneedles, e.g., as shown in Fig. 3. The substance transfer component can be or include, as described elsewhere herein and in other documents incorporated herein by reference, any of a variety of components able to receive a substance from the skin and/or from beneath the skin of a subject, and/or deliver a substance to the skin and/or to a location beneath the skin of the subject. For example, the substance transfer component may include one or more needles and/or microneedles, a hygroscopic agent, a cutter or other piercing element, an electrically-assisted system, or the like. In the specific device illustrated, substance transfer component 1108 includes an array of skin insertion objects such as solid or hollow microneedles. In one set of embodiments, substance transfer component 1108 is selected to have a particular size and profile for a particular use. For example, the substance transfer component may include an array of skin insertion objects which, in the device illustrated, emanate from a base 1110 which will be described further below.

In certain embodiments, a plurality of skin insertion objects of the substance transfer component 1108 and are relatively small, and are relatively completely driven into the skin. The skin insertion objects may be positioned to address the skin of the subject, each protruding from a base and defining a length from the base, and are able to be inserted into or through the skin to a depth essentially equal to their length but are prevented, by the base, from inserting at a depth greater than their length. In some embodiments, the plurality of skin insertion objects have an average length (measured from the base) of no more than about 1,000 microns or more than about 2,000 microns, although lengths can differ between individual skin insertion objects. In one set of embodiments, the skin insertion objects are of relatively uniform length, together defining an average length and each differing from the average length by no more than about 50%, about 40%, about 30%, about 10%, or about 5%. The average length of the skin insertion objects, in other embodiments, are no more than about 1,500 microns, no more than about 1,000 microns, no more than about 900 microns, no more than about 800 microns, no more than about 750 microns, no more than about 600 microns, no more than about 500 microns, no more than about 400 microns, or no more than about 350 microns. In some embodiments, a deployment actuator as discussed herein is provided that is able to move the skin insertion objects from a fully pre-deployed position to a fully deployed position with a force sufficient to insert the plurality of skin insertion object into or through the skin to an average insertion depth of at least about 50% the average length of the plurality of skin insertion objects. In other embodiments, the deployment actuator is able to insert the plurality of skin insertion objects to an average insertion depth of at least about 55%, at least about 60%, at least about 65%, at least about 70%, at least about 75%, at least about 80%, at least about 85%, at least about 90%, at least about 92%, about 94%, about 96%, or about 98% of the average length of the plurality of skin insertion objects.

In the device illustrated, the skin insertion objects of the substance transfer component 1108 are mounted on a flexible structure 1112 which, as illustrated, is maintained relatively rigidly through various aspects of the device but which mounts substance transfer component 1108 flexibly for up/down movement relative to the skin. Flexible structure 1112 can be a membrane, a single or multi-layer structure selected from various polymers or the like to provide sufficient properties such as any combination of flexibility, elasticity, gas permeability or impermeability, fluid permeability or impermeability, or the like for desired operation. Portions of flexible structure 1112, skin insertion objects 1108, and other interior walls of the device define a region 1114 which allows for movement of skin insertion objects 1108 relative to the skin for delivery of a substance to and/or receiving of a substance from the skin or beneath the skin, and, where a substance is received from the skin or from beneath the skin, region 1114 can serve as a reservoir for introduction of the substance into the device. Where a vacuum is used to receive a substance from the subject (as in the embodiment illustrated in Fig. 3), region 1114, when positioned against the skin, can expose vacuum to that portion of the skin proximate surface 1104 of the device and abutting the chamber.

Device 1100 also includes a device actuator 1116 which, as illustrated, includes a proximate portion 1118 which can be addressed by a user of the device (who may be the same or different from the subject the device is administered to) and a distal portion 1120 for addressing skin insertion objects 1108 via flexible structure 1112. Proximal portion 1118 and distal portion 1120 are, in the device illustrated, opposite ends of a single component but, as would be understood by those of ordinary skill in the art, the actuator can include a plurality of individual components operably linked in any way necessary to perform actuation as will be described.

As will be understood, Fig. 3 is a cross-section of a device illustrating various components and channels within the device. As will also be understood by those of ordinary skill in the art, different arrangements of devices and channels are contemplated herein so long as the purpose of the device described herein is met. In this figure, device actuator 1116 is directly connected to or otherwise operably linked to a deployment actuator 1122 which, in the device illustrated, is in the form of a "snap dome," the function and use of which will be described below. The snap dome in this figure has an approximately circular profile. The structure may define an interior and a periphery which, if not circular, may include a plurality of tabs, protrusions, or the like sufficient for support of structure 1122 within the device. As illustrated, a plurality of tabs (or the essentially circular perimeter of) the device are supported within holders 1124, and the center, snap dome portion of the device is operably linked to device actuator 1116, such that movement of the central portion of snap dome 1122 and the periphery of the snap dome can be controlled independently of each other. Holders 1124 are directly connected to or otherwise operably linked to a retraction actuator 1126 which, in the device illustrated, can be a ring-shaped structure positioned under and supporting holders 1124. Holders 1124 can be individual holders and/or a ring-like structure surrounding the periphery of snap dome 1122. A series of one, two, or more support members (e.g., 1130) are positioned near the top of device 1100 and serve to define a series of channels for sample flow, vacuum control, or the like as will be described.

Turning now to channels defined within the device, as described above, region 1114, when the device is positioned against skin, can serve to expose a portion of the skin defined by the periphery of the region to a vacuum, to substance transfer component 1108 as it moves toward and/or away from the skin, and/or to transfer a substance from or to the subject. Region 1114 can house a substance for transfer to the subject, in the form of a pharmaceutical composition or the like, optionally loaded on skin insertion objects 1108. Where blood and/or interstitial fluid is drawn from a subject, region 1114 can serve to introduce the substance into the device from the subject.

A channel 1132 connects region 1114 to other portions of the device in this example. Channel 1132 can be used to deliver a substance to region 1114 for transfer to a subject, or for application of a vacuum to region 1114, and/or for receiving of a substance from a subject. The remainder of the description of device 1100 will be made within the context of receiving a substance such as blood and/or interstitial fluid from a subject, but it is to be understood that substances can also be delivered via various channels. Channel 1132 typically emanates in one direction from region 1114 although a plurality of channels can emanate from the region, arranged radially or otherwise relative to the center of the device. In device 1100, channel 1132 first passes laterally from the center of the device and then upwardly where, near the top of the device, it can, optionally, include one wall defining a window 1134 through which a user of the device can observe transfer of a substance, or through which analysis of a substance may occur. It can also itself define a reservoir, in whole or in part, or be connected to an internal or an external reservoir for maintaining, storing, and/or transferring a substance drawn from a subject. As shown here, it can be connected to a substance collection reservoir 1136 which, as illustrated, is a disc-shaped reservoir formed in the device housing and surrounding the center of the device including device actuator 1116 and related components.

Device 1100, illustrated as one example of devices provided by the invention, includes a vacuum chamber for applying a vacuum proximate the skin of a subject for receiving a substance from the skin. As illustrated, vacuum chamber 1138 is positioned in a central portion of the device surrounding device actuator 1116, although it can be provided anywhere in or proximate the device. The vacuum chamber can be evacuated to an appropriate level just prior to use, or the device can be pre-packaged under vacuum as described elsewhere herein. As illustrated, vacuum chamber 1138 is in fluid communication with substance collection reservoir 1136 but, in its initial state and prior to use, a membrane or other component, such as support member 1128, separates channel 1132 connecting it to region 1102. In the device illustrated, a vacuum actuation component 1140 can be actuated to puncture the membrane or other component (e.g., 1128) and thereby connect vacuum chamber 1138 with channel 1132, at an appropriate time during use of the device. In other embodiments, device actuator 1116 and vacuum actuation component 1140 can be combined into a single button or operably linked so that only one operation is needed to actuate both the skin insertion objects and the vacuum.

Deployment actuator (or, as shown, a snap dome) 1122 can be provided in a variety of forms including a monostable or bistable configuration. In the embodiment illustrated, a bistable configuration is illustrated including first and second low energy or stable configurations separated by a relatively high energy or unstable configuration. As shown, the deployment actuator 1122 is shown in a "cocked" or pre-deployed position.

The deployment actuator may be formed from any suitable material, for example, a metal such as stainless steel (e.g., 301, 301LN, 304, 304L, 304LN, 304H, 305, 312, 321, 321H, 316, 316L, 316LN, 316Ti, 317L, 409, 410, 430, 440A, 440B, 440C, 440F, 904L), carbon steel, spring steel, spring brass, phosphor bronze, beryllium copper, titanium, titanium alloy steels, chrome vanadium, nickel alloy steels (e.g., Monel 400, Monel K 500, Inconel 600, Inconel 718, Inconel x 750, etc.), a polymer (e.g., polyvinylchloride, polypropylene, polycarbonate, etc.), a composite or a laminate (e.g., comprising fiberglass, carbon fiber, bamboo, Kevlar, etc.), or the like. The deployment actuator may be of any shape and/or size. For example, the deployment actuator may have a generally domed shape (e.g., as in a snap dome), and be circular (no legs), or the deployment actuator may have other shapes, e.g., oblong, triangular (3 legs), square (4 legs), pentagonal (5 legs), hexagonal (6 legs), spiderlegged, starlike, clover-shaped (with any number of lobes, e.g., 2, 3, 4, 5, etc.), or the like. The deployment actuator may have, in some embodiments, a hole, dimple, or button in the middle. The deployment actuator may also have a serrated disc or a wave shape. In some cases, the substance transfer component may be mounted on the deployment actuator. In other cases, however, the substance transfer component is mounted on a separate structure which is driven or actuated upon movement of the deployment actuator.

In one set of embodiments, the deployment actuator is not planar, and has a portion that can be in a first position (a "cocked" or pre-deployed position) or a second position (a "fired" or deployed position), optionally separated by a relatively high energy configuration. In some cases, the pre-deployed position may be at a higher energy level than the deployed position. In some cases, both the first position and the second position are stable (i.e., the structure is bistable), although conversion between the first position and the second position requires the structure to proceed through an unstable configuration.

In some cases, surprisingly, the distance or separation between the first position and the second position is relatively small. Such distances or separations may be achieved using snap domes or other configurations such as those described herein, in contrast to springs or other devices which require longer translational or other movements. For example, the perpendicular distance (i.e., in a direction away from the skin) in the deployment actuator between the top of the structure and the bottom of the structure (excluding the substance transfer component) when the device containing the structure is placed on the skin of a subject may be no more than about 5 mm, no more than about 4 mm, no more than about 3 mm, no more than about 2 mm, no more than about 1 mm in some cases, no more than about 0.8 mm, no more than about 0.5 mm, or no more than about 0.3 mm. In one set of embodiments, the distance is between about 0.3 mm and about 1.5 mm. In another set of embodiments, the deployment actuator may have a greatest lateral dimension (parallel to the skin) when the device containing the structure is placed on the skin of a subject of no more than about 50 mm, no more than about 40 mm, no more than about 30 mm, no more than about 25 mm, no more than about 20 mm, no more than about 15 mm, no more than about 5 mm, no more than about 4 mm, no more than about 3 mm, no more than about 2 mm, no more than about 1 mm in some cases, no more than about 0.8 mm, no more than about 0.5 mm, or no more than about 0.3 mm. In one set of embodiments, the distance is between about 0.3 mm and about 1.5 mm.

Use of device 1100 will now be described in the context of receiving a substance such as blood from a subject. Device 1100 is placed against the skin of a subject such that at least a portion of surface 1104 contacts the skin. Prior to use, a cover member (not shown) can cover surface 1104 of the device and can cover region 1114, to protect surface 1104 and region 1114 from contaminants, etc. optionally maintaining the interior of the device in a sterile condition. The cover can be peeled off or otherwise removed from the device, and the device placed against the skin, optionally adhering to the skin. Vacuum actuation component 1140 can be actuated to expose channel 1132 and region 1114 to vacuum at any time, including before, simultaneously, or after actuation of substance transfer component 1108. In one arrangement, vacuum actuation component 1140 is actuated to apply vacuum to region 1114 prior to actuation to substance transfer component 1108, thereby to create a vacuum against the skin proximate region 1114 prior to use. Actuation of device actuator 1116 can take place before or after deployment of vacuum.

When device actuator 1116 is actuated by a user (e.g., when proximal portion 1118 is depressed downwardly as shown in the figure), distal portion 1120 engages skin insertion objects 1108 (optionally via flexible structure 1112) to drive it toward the skin. In some embodiments, foil 1128 is first broken, then retraction actuator 1126 is compressed, then retraction actuator 1126 is broken, before flexible structure 1112 is stretched and the deployment actuator 1122 of the device fires or is actuated. Membranes or other members 1112, 1128, or 1130 may have, in some cases, sufficient flexibility and/or elasticity to allow actuator 1116 to drive skin insertion objects 1108 sufficiently distally (downwardly, as shown) to engage the skin of the subject and carry out the desired function of the device. Various gaskets, bearings, or membranes as shown can be used for this function. Where support member 1128 is a foil or the like used for the purpose of initially separating vacuum reservoir 1138 from channel 1132 (e.g., prior to use), when device actuator 1116 is moved downwardly, vacuum actuation component 1140 may rupture support member 1128 proximate actuator 1116, or flexibly deform as need be, so long as member 1130 (or another component) serves to allow device actuator 1116 to move slidably within the device while maintaining sufficient vacuum in vacuum reservoir 1138 and related channels for use of the device.

When skin insertion objects 1108 engage the skin of the subject and facilitates receiving of a substance from the skin and/or from beneath the skin of the subject, a vacuum can draw the substance into region 1114, through channel or channels 1132, and into substance collection reservoir 1136. In this process, device actuator 1116 first urges deployment actuator 1122 from its first stable configuration to a relatively unstable configuration and beyond that point, at which point the deployment actuator 1122 rapidly moves to a second stable configuration associated with downward driving of device actuator 1116 to quickly drive access substance transfer component 1108 proximate the skin.

After that point, if it is desirable for access substance transfer component 1108 to be received from the skin, then a variety of techniques can be used to do so. In the device illustrated, retraction actuator 1126 drives holder 1124 upwardly, retracting structure 1122 and device actuator 1116 from substance transfer component 1108. At that point, device actuator 1116 can be operably linked to transfer component 1108 and retract the transfer component, or it can move freely relative to substance transfer component 1108, whereby flexible structure 1112 (e.g., an elastic membrane) or other component can retract substance transfer component 1108 from the skin. The retraction actuator 1126 may include any suitable retraction component. Again, in the device illustrated, retraction actuator 1126 can itself be a reversibly deformable structure such as a leaf spring, coil spring, foam, or the like. During use, when device actuator 1116 is driven downwardly, retraction actuator 1126 is first compressed and, depending upon the size and arrangement of components 1126, 1124, 1122, 1116 and 1108, during compression, substance transfer component 1108 can be driven downwardly to some extent. At the point at which retraction actuator 1126 is compressed and provides a sufficient resistance force, deployment actuator 1122 can be urged from its first configuration through an unstable configuration and can return to its second configuration, driving substance transfer component 1108 against the skin. Then, upon release of user pressure (or other actuation, which can be automatic) from actuator 1116, retraction actuator 1126 can expand and, with structure 1122 optionally remaining in its second, downwardly-driven low-energy configuration, actuator 1116 can be retracted and substance transfer component 1108 retracted from the skin.

In one set of embodiments, the deployment actuator may move a substance transfer component from a fully pre-deployed position to a fully deployed position in which the substance transfer component is fully engaged with the skin, in a short period of time. In one embodiment, that period of time is less than about 0.01 seconds, and in other embodiments, less than about 0.009 seconds, less than about 0.008 seconds, less than about 0.007 seconds, less than about 0.006 seconds, less than about 0.005 seconds, less than about 0.004 seconds, less than about 0.003 seconds, less than about 0.002 seconds, less than about 0.001 seconds, less than about 0.0005 seconds, less than about 0.00025, or less than about 0.0001 seconds.

In some cases, the substance transfer component achieves relatively high accelerations due to the deployment actuator. For example, in some cases, the deployment actuator can produce average accelerations (e.g., average acceleration from start of movement to a position where a substance transfer component first contacts a subject) of at least about 4 m/s², about 6 m/s², about 8 m/s², about 10 m/s², about 12 m/s², about 15 m/s², or about 20 m/s², at least about 30 m/s², at least about 50 m/s², at least about 100 m/s², at least about 300 m/s², at least about 500 m/s², at least about 1,000 m/s², at least about 3,000 m/s², at least about 5,000 m/s², at least about 10,000 m/s², at least about 30,000 m/s², at least about 50,000 m/s², at least about 60,000 m/s², at least about 70,000 m/s², at least about 100,000 m/s², at least about 200,000 m/s², or at least about 300,000 m/s². In some cases, the deployment actuator can produce instantaneous accelerations of at least about 4 m/s², about 6 m/s², about 8 m/s², about 10 m/s², about 12 m/s², about 15 m/s², or about 20 m/s², at least about 30 m/s², at least about 50 m/s², at least about 100 m/s², at least about 300 m/s², at least about 500 m/s², at least about 1,000 m/s², at least about 3,000 m/s², at least about 5,000 m/s², at least about 10,000 m/s², at least about 30,000 m/s², at least about 50,000 m/s², at least about 60,000 m/s², at least about 70,000 m/s², at least about 80,000 m/s², at least about 80,000 m/s², at least about 100,000 m/s², at least about 200,000 m/s², or at least about 300,000 m/s².

Average acceleration is used to mean the rate of change of velocity over the entire time period from the pre-deployed position to the deployed position, and instantaneous acceleration is used to mean the acceleration at a specific point in time during the time period. The average acceleration and the instantaneous accelerations may be determined using high-speed imaging analysis. High speed imaging is used to capture a sequence of video frames with very short time steps between frames. In one example, the time step between frames may be 66 microseconds, although other time steps are possible depending on imaging equipment capability and/or the total capture time. Each video frame captures the position of a moving object at a specific moment in time. Using this data, position as a function of time may be plotted. An equation that is fit to this data approximates position as a function of time. The second derivative of the position equation is an equation for instantaneous acceleration as a function of time. The acceleration equation can then be used to calculate the instantaneous acceleration at any given point in time.

In some embodiments, the substance transfer component is accelerated for relatively short periods of time, e.g., less than about 1 second, less than about 300 milliseconds, less than about 100 milliseconds, less than about 30 milliseconds, less than about 10 milliseconds, less than about 3 milliseconds, or less than about 1 millisecond, and/or over relatively short distances, e.g., less than about 5 millimeters, less than about 4 millimeters, less than about 3 millimeters, less than about 2 millimeters, less than about 1 millimeter, less than about 800 micrometers, less than 600 micrometers, less than 500 micrometers, less than 400 micrometers, less than about 300 micrometers, less than about 200 micrometers, less than about 100 micrometers, less than about 50 micrometers, etc. Significant forces can be applied to the substance transfer component as it moves relative to the skin via the deployment actuator. In another set of embodiments, the substance transfer component, at the point at which it first contacts the skin, is driven by a force created at least in part by the deployment actuator of at least about 6 micronewtons, about 8 micronewtons, about 10 micronewtons, about 12 micronewtons, or about 15 micronewtons.

Devices of the invention can provide significant advantage in some embodiments. For example, deployment actuators able to move substance transfer components in short time periods, and/or at high velocities, and/or with high forces, and/or with high pressure, and/or drive relatively short substance transfer components such as skin insertion objects or microneedles relatively deeply into the skin and/or through the skin, and/or any combination of the above can provide significant advantage. In some embodiments, these features can provide better control of substance delivery or receipt. Better mechanical stability can be provided in some cases by shorter substance transfer components (e.g., bending and/or buckling can be avoided) and relatively shorter substance transfer components , designed to be driven relatively completely (for example, through nearly all of their entire length) into the skin may offer better control of penetration in some embodiments. If better control of penetration can be achieved, better delivery or receiving can also be achieved in some cases, for example, resulting in less pain or essentially painless deployment.

Moreover, if substance transfer components are used to deliver a substance such as a pharmaceutical composition into or through the skin, more precise delivery can be provided, according to certain embodiments. With better, precise control over depth of insertion of the substance transfer components (e.g., by using devices designed to insert the substance transfer components essentially fully), and/or the substance transfer components contain and/or are coated with a pharmaceutical composition, then more control exists over the amount of pharmaceutical substance inserted into the skin by the substance transfer components, in some embodiments. Furthermore, quick and/or high velocity, and/or high force and/or pressure application of skin insertion objects to the skin may in certain embodiments result in lower pain or painless deployment.

In one set of embodiments, devices such as those described herein may be used in other organs of the body, besides the skin. Accordingly, it should be understood that the descriptions above (e.g., by way of skin insertion objects, substance transfer components, fluid transporters, etc., and devices able to be applied to the skin) and elsewhere in this application are by way of example only, and in other embodiments, any such devices may also be applied to an organ of the body instead of and/or in addition to the skin. For example, another set of embodiments of the invention involve substance transfer components, insertion objects or fluid transporters, including needles or microneedles, that may be applied to and/or inserted into an organ within a subject, not just the skin, to deliver and/or receive a fluid such as blood to the organ. For instance, the device may be applied to an organ to receive a substance from a subject, for example, blood or another bodily fluid, such as cerebrospinal fluid, gastric fluid, lymph, etc., and/or the device may be used to deliver a substance to the organ, e.g., to the entire organ, or to only a portion of the organ. In some embodiments, the device may be used to take a solid (or semi-solid) sample of tissue. For example, the device may take a biopsy of an organ. For instance, the device may scrape off a portion of the organ, or cut out a portion of the organ. Examples of organs from which substances may be delivered to and/or received from include, but are not limited to, the brain, the breast, a kidney, a lymph node, a lung, bone marrow, a portion of the gastrointestinal tract, the liver, the skin, a lesion, a tumor, or the like.

Scraping may comprise, in one set of embodiments, a reciprocating action whereby an instrument of the device is scraped along the surface of the organ in one, two, or more directions. Scraping can also be accomplished by a rotating action, for example parallel to the surface of the organ and in one direction (i.e., with a roller drum) or parallel to the surface of the organ and in a circular manner (i.e., with a drilling instrument). A cutting mechanism may comprise a blade capable of making one or more incisions and a mechanism for removing a portion of tissue (i.e., by suction or mechanically picking up) or may use a pincer mechanism for cutting out a portion of tissue. A cutting mechanism may also function by a coring action. For example, a hollow cylindrical device can be penetrated into the organ such that a cylindrical core of tissue may be removed.

According to one set of embodiments, many devices as discussed herein use various techniques for delivering and/or receiving fluid, for example, in connection with substance transfer components, skin insertion objects, or the like. For example, one or more needles and/or microneedles, a hygroscopic agent, a cutter or other piercing element, an electrically-assisted system, or the like may be used in conjunction with a snap dome or other device as described above. Additional examples of such techniques are described herein and/or in the applications incorporated herein. It is to be understood that, generally, fluids may be delivered and/or received in a variety of ways, and various systems and methods for delivering and/or receiving fluid from the skin (or other organs) are discussed below and/or in the applications incorporated herein. In some embodiments, for example, techniques for piercing or altering the surface of the skin to transport a fluid are discussed, for example, using a needle such as a hypodermic needle or microneedles, chemicals applied to the skin (e.g., penetration enhancers), jet injectors or other techniques such as those discussed below, etc.

As an example, in one embodiment, a needle such as a hypodermic needle can be used to deliver and/or receive fluid to or from the skin or other organ. Hypodermic needles are well-known to those of ordinary skill in the art, and can be obtained commercially with a range of needle gauges. For example, the needle may be in the 20-30 gauge range, or the needle may be 32 gauge, 33 gauge, 34 gauge, etc.

If needles are present, the needles may be of any suitable size and length, and may be solid or hollow. The needles may have any suitable cross-section (e.g., perpendicular to the direction of penetration), for example, circular, square, oval, elliptical, rectangular, rounded rectangle, triangular, polygonal, hexagonal, irregular, etc. For example, the needle may have a length of less than about 5 mm, less than about 4 mm, less than about 3 mm, less than about 2 mm, less than about 1 mm, less than about 800 micrometers, less than 600 micrometers, less than 500 micrometers, less than 400 micrometers, less than about 300 micrometers, less than about 200 micrometers, less than about 175 micrometers, less than about 150 micrometers, less than about 125 micrometers, less than about 100 micrometers, less than about 75 micrometers, less than about 50 micrometers, etc. The needle may also have a largest cross-sectional dimension of less than about 5 mm, less than about 4 mm, less than about 3 mm, less than about 2 mm, less than about 1 mm, less than about 800 micrometers, less than 600 micrometers, less than 500 micrometers, less than 400 micrometers, less than about 300 micrometers, less than about 200 micrometers, less than about 175 micrometers, less than about 150 micrometers, less than about 125 micrometers, less than about 100 micrometers, less than about 75 micrometers, less than about 50 micrometers, etc. For example, in one embodiment, the needle may have a rectangular cross section having dimensions of 175 micrometers by 50 micrometers. In one set of embodiments, the needle may have an aspect ratio of length to largest cross-sectional dimension of at least about 2:1, at least about 3:1, at least about 4:1, at least 5:1, at least about 7:1, at least about 10:1, at least about 15:1, at least about 20:1, at least about 25:1, at least about 30:1, etc.

In one embodiment, the needle is a microneedle. As an example, microneedles such as those disclosed in U.S. Patent No. 6,334,856, issued January 1, 2002, entitled "Microneedle Devices and Methods of Manufacture and Use Thereof," by Allen, *et al.,* may be used to deliver and/or receive fluids or other materials to or from a subject. The microneedles may be hollow or solid, and may be formed from any suitable material, e.g., metals, ceramics, semiconductors, organics, polymers, and/or composites. Examples include, but are not limited to, pharmaceutical grade stainless steel, titanium, nickel, iron, gold, tin, chromium, copper, alloys of these or other metals, silicon, silicon dioxide, and polymers, including polymers of hydroxy acids such as lactic acid and glycolic acid polylactide, polyglycolide, polylactide-co-glycolide, and copolymers with polyethylene glycol, polyanhydrides, polyorthoesters, polyurethanes, polybutyric acid, polyvaleric acid, polylactide-co-caprolactone, polycarbonate, polymethacrylic acid, polyethylenevinyl acetate, polytetrafluorethylene, polymethyl methacrylate, polyacrylic acid, or polyesters.

In some cases, more than one microneedle may be used. For example, arrays of microneedles may be used, and the microneedles may be arranged in the array in any suitable configuration, e.g., periodic, random, etc. In some cases, the array may have 3 or more, 4 or more, 5 or more, 6 or more, 10 or more, 15 or more, 20 or more, 35 or more, 50 or more, 100 or more, or any other suitable number of microneedles. In some embodiments, the device may have at least 3 but no more than 5 needles or microneedles (or other skin insertion objects), at least 6 but no more than 10 needles or microneedles, or at least 11 but no more than 20 needles or microneedles. Typically, a microneedle will have an average cross-sectional dimension (e.g., diameter) of less than about a micron. It should be understood that references to "needle" or "microneedle" as discussed herein are by way of example and ease of presentation only, and that in other embodiments, more than one needle and/or microneedle may be present in any of the descriptions herein.

Those of ordinary skill in the art can arrange needles relative to the skin for these purposes including, in one embodiment, introducing needles into the skin at an angle, relative to the skin's surface, other than 90°, i.e., to introduce a needle or needles into the skin in a slanting fashion so as to limit the depth of penetration. In another embodiment, however, the needles may enter the skin at approximately 90°.

In one set of embodiments, an array of needles or microneedles, or other substance transfer components, may be used. For example, the needles (or other objects) may be arranged in any suitable configuration, e.g., linear, periodic, random, etc. In some embodiments, all of the needles (or other objects) may be used simultaneously, e.g., to deliver and/or receive fluid or other substance to or from a subject. In other embodiments, however, only a portion of the needles may be used per use, e.g., one needle (or a first set of needles) may be used at a first time, while another needle (or a second set of needles) may be used at a second time. The needles may be actuated individually in some cases, e.g., a first needle is brought to the skin at a first time, while a second needle is brought to the skin at a second time. In one set of embodiments, the needles (or other objects) may be positioned on a rotatable structure, e.g., that rotates between uses so that fresh needles are ready to be brought to the skin (or other organ) each time. For instance, the needles may be attached to a "carousel" so that one or more needles from the carousel may be brought to the skin when needed, e.g., to deliver and/or receive fluid to or from a subject. In some cases, the array of needles or microneedles may be present on a module that can be replaced, e.g., without necessarily needing to replace the entire device. For instance, after use, the module may be ejected from the device and optionally thrown away, and replaced with a fresh module.

In some cases, the microneedles may be present in an array selected such that the density of microneedles within the array is between about 0.5 needles/mm² and about 10 needles/mm², and in some cases, the density may be between about 0.6 needles/mm² and about 5 needles/mm², between about 0.8 needles/mm² and about 3 needles/mm², between about 1 needles/mm² and about 2.5 needles/mm², or the like. In some cases, the needles may be positioned within the array such that no two needles are closer than about 1 mm, about 0.9 mm, about 0.8 mm, about 0.7 mm, about 0.6 mm, about 0.5 mm, about 0.4 mm, about 0.3 mm, about 0.2 mm, about 0.1 mm, about 0.05 mm, about 0.03 mm, about 0.01 mm, etc.

The needles or microneedles may have any suitable length, and the length may be, in some cases, dependent on the application. For example, needles designed to only penetrate the epidermis may be shorter than needles designed to also penetrate the dermis, or to extend beneath the dermis or the skin. In certain embodiments, the needles or microneedles may have a maximum penetration into the skin, or insertion depth, of no more than about 3 mm, no more than about 2 mm, no more than about 1.75 mm, no more than about 1.5 mm, no more than about 1.25 mm, no more than about 1 mm, no more than about 900 microns, no more than about 800 microns, no more than about 750 microns, no more than about 600 microns, no more than about 500 microns, no more than about 400 microns, no more than about 300 microns, no more than about 200 microns, no more than about 175 micrometers, no more than about 150 micrometers, no more than about 125 micrometers, no more than about 100 micrometers, no more than about 75 micrometers, no more than about 50 micrometers, etc. In certain embodiments, the needles or microneedles may be selected so as to have a maximum insertion depth of at least about 50 micrometers, at least about 100 micrometers, at least about 300 micrometers, at least about 500 micrometers, at least about 1 mm, at least about 2 mm, at least about 3 mm, etc.

In one set of embodiments, the needles (or microneedles) may be coated. For example, the needles may be coated with a substance that is delivered when the needles are inserted into the skin. For instance, the coating may comprise heparin, an anticoagulant, an anti-inflammatory compound, an analgesic, an anti-histamine compound or a vasodilator to assist with the flow of blood from the skin of the subject. The coating may comprise a drug or other therapeutic agent such as those described herein. The drug or other therapeutic agent may be one used for localized delivery (e.g., of or proximate the region to which the coated needles or microneedles are applied), and/or the drug or other therapeutic agent may be one intended for systemic delivery within the subject.

At least some the skin insertion objects may be at least partially coated by a substance such as a drug, analgesic or agent by using dip or spray coating or other suitable technique. Thus, the substance may be delivered to the skin by the substance dissolving or otherwise detaching from the substance transfer component at or in the skin or other subject site. Alternately, the substance may be delivered after a substance transfer component penetrates the subject, e.g., in a way similar to a hypodermic needle. For example, a skin insertion object of the substance transfer component may be inserted into the skin, and a substance may be pumped or pushed through a hole, groove or other channel of the skin insertion object (e.g., by a high pressure gas).

A drug may be any composition which possesses therapeutic, prophylactic, or diagnostic properties *in vivo,* for example when administered to an animal, including mammals, such as humans. The drug can be for local treatment or for regional or systemic therapy. The drug can be or include a peptide, protein, carbohydrate (including monosaccharides, oligosaccharides, and polysaccharides), nucleoprotein, mucoprotein, lipoprotein, glycoprotein, nucleic acid molecules (including any form of DNA such as cDNA, RNA, or a fragment thereof, oligonucleotides, and genes), nucleotide, nucleoside, lipid, biologically active organic or inorganic molecules, or combination thereof. Examples of suitable therapeutic and/or prophylactic active agents include anti-infectives, analgesics, antiinflammatories, steroids, decongestants, neuroactive agents, anesthetics, and sedatives. Examples of suitable diagnostic agents include radioactive isotopes and radioopaque agents, metals, gases, labels including chromatographic, fluorescent, or enzymatic labels.

Examples of biologically active polypeptides or proteins include, but are not limited to, glucagon, glucagon-like peptides such as, GLP-1, GLP-2 or other GLP analogs, derivatives or agonists of Glucagon Like Peptides, exendins such as, exendin-3 and exendin-4, derivatives, agonists and analogs thereof, vasoactive intestinal peptide (VIP), immunoglobulins, antibodies, cytokines (e.g., lymphokines, monokines, chemokines), interleukins, macrophage activating factors, interferons, erythropoietin, nucleases, tumor necrosis factor, colony stimulating factors (e.g., G-CSF), insulin, enzymes (e.g., superoxide dismutase, plasminogen activator, etc.), tumor suppressors, blood proteins, hormones and hormone analogs and agonists (e.g., follicle stimulating hormone, growth hormone, adrenocorticotropic hormone, and luteinizing hormone releasing hormone (LHRH)), vaccines (e.g., tumoral, bacterial and viral antigens), antigens, blood coagulation factors, growth factors (NGF and EGF), gastrin, GRH, antibacterial peptides such as defensin, enkephalins, bradykinins, calcitonin and muteins, analogs, truncation, deletion and substitution variants and pharmaceutically acceptable salts of all the foregoing. Suitable analgesics include but are not limited to lidocaine, bupivacaine, and tetracaine. Suitable steroids include but are not limited to cortisone, betametasone, budesonide and fluticasone.

In one set of embodiments, the needles or microneedles may be used to deliver a drug into the skin of a subject. The needles or microneedles may be at least partially coated, and the coating may comprise a drug or other therapeutic agent such as those described herein. For example, in one set of embodiments, at least about 50%, at least about 60%, at least about 70%, at least about 80%, at least about 90%, or substantially all of a needle or a microneedle may be coated, and one or more than one needle or microneedle may be coated in a device as discussed herein. For instance, at least about 25%, at least about 50%, at least about 60%, at least about 70%, at least about 80%, at least about 90%, or substantially all of the needles or microneedles in a device may comprise a coating.

Without wishing to be bound by any theory, it is believed that, at least in some cases, longer needles or microneedles may be useful for the delivery of a drug or other therapeutic agent. For example, a needle having a greater depth of penetration into the skin may be useful for delivering the drug or other therapeutic agent deeper into the skin, e.g., closer to capillaries within or below the skin, which may minimize the distance the drug needs to travel before being available systemically and allow a more rapid onset of the drug effect. In addition, greater depth of penetration can be useful for delivering greater amounts of drug. A longer needle can have more surface area exposed internally of the subject, relative to a shorter needle (e.g., of the same diameter), and the increased surface area may allow more of the coating containing drug to be exposed internally of the skin. Thus, for example, a greater amount of drug may be delivered per needle or microneedle that enters the skin.

Accordingly, in certain embodiments, relatively long needles or microneedles may be used for the delivery of a drug or other therapeutic agent into the skin, for example. For instance, the average length of the needles or microneedles in the device may be at least about 200 micrometers, at least about 300 micrometers, at least about 400 micrometers, at least about 500 micrometers, at least about 600 micrometers, at least about 750 micrometers, at least about 800 micrometers, at least about 900 micrometers, at least about 1,000 micrometers, at least about 1,200 micrometers, at least about 1,500 micrometers, at least about 1,700 micrometers, or at least about 2,000 micrometers in some embodiments.

Any of a variety of suitable techniques may be used to coat a needle or a microneedle. For instance, the needle or microneedle may be coated by exposing the needles or microneedles to a liquid containing a substance to be coated thereon. For example, the needles or microneedles may be dipped into a liquid, a liquid may be sprayed on or aerosolized onto the needles or microneedles, an electric field may be used to attract a charged liquid onto the needles or microneedles, etc.

In one embodiment, the fluid is delivered and/or received manually, e.g., by manipulating a plunger on a syringe. In another embodiment, the fluid can be delivered and/or received from the skin mechanically or automatically, e.g., using a piston pump or the like. Fluid may also be received using vacuums such as those discussed herein. For example, vacuum may be applied to a conduit, such as a needle, in fluidic communication with a bodily fluid in order to draw up at least a portion of the fluid from the pooled region. In yet another embodiment, fluid is received using capillary action (e.g., using a microfluidic channel or hypodermic needle having a suitably narrow inner diameter). In still another embodiment, pressure may be applied to force fluid out of the needle.

In some embodiments, a substance is delivered to a subject from a device. In cases where the needle or other skin insertion object is coated with a drug or other substance, the device may deliver the drug or substance to a subject by penetrating the skin with the coated needle. The substance may be delivered to or beneath the skin by the substance dissolving or otherwise detaching from the substance transfer component at the skin or other subject site. The device may or may not cause fluid release from the subject. In some cases, fluid from the subject is not received into the device and a vacuum source is not needed. Also, in some cases, the device may additionally or alternatively deliver a fluid drug or other fluid substance to a subject. The fluid substance may be delivered to or beneath the skin through hollow needles that transfer fluid from the device to the subject.

As still another example, pressurized fluids may be used to deliver fluids or other materials into the skin, for instance, using a jet injector or a "hypospray." Typically, such devices produce a high-pressure "jet" of liquid or powder (e.g., a biocompatible liquid, such as saline) that drives material into the skin, and the depth of penetration may be controlled, for instance, by controlling the pressure of the jet. The pressure may come from any suitable source, e.g., a standard gas cylinder or a gas cartridge. A non-limiting example of such a device can be seen in U.S. Patent No. 4,103,684, issued August 1, 1978, entitled "Hydraulically Powered Hypodermic Injector with Adapters for Reducing and Increasing Fluid Injection Force," by Ismach. Pressurization of the liquid may be achieved, for example, using compressed air or gas, for instance, from a gas cylinder or a gas cartridge.

In some embodiments, fluid may be received using a hygroscopic agent applied to the surface of the skin, or proximate the skin. For example, a device as described herein may contain a hygroscopic agent. In some cases, pressure may be applied to drive the hygroscopic agent into the skin. Hygroscopic agents typically are able to attract water from the surrounding environment, for instance, through absorption or adsorption. Non-limiting examples of hygroscopic agents include sugar, honey, glycerol, ethanol, methanol, sulfuric acid, methamphetamine, iodine, many chloride and hydroxide salts, and a variety of other substances. Other examples include, but are not limited to, zinc chloride, calcium chloride, potassium hydroxide, or sodium hydroxide. In some cases, a suitable hygroscopic agent may be chosen based on its physical or reactive properties, e.g., inertness or biocompatibility towards the skin of the subject, depending on the application.

In some embodiments, the device may comprise a cutter able to cut or pierce the surface of the skin. The cutter may comprise any mechanism able to create a path through which fluids may be delivered and/or received from the skin. For example, the cutter may comprise a hypodermic needle, a blade (e.g., a knife blade, a serrated blade, etc.), a piercing element (e.g., a lancet or a solid or a hollow needle), or the like, which can be applied to the skin to create a suitable conduit for the delivery and/or receiving of fluid from the skin. In one embodiment, a cutter is used to create such a pathway and removed, then fluid may be delivered and/or received via this pathway. In another embodiment, the cutter remains in place within the skin, and fluid may be delivered and/or received through a conduit within the cutter.

In some embodiments, fluid may be received using an electric charge. For example, reverse iontophoresis may be used. Without wishing to be bound by any theory, reverse iontophoresis uses a small electric current to drive charged and highly polar compounds across the skin. Since the skin is negatively charged at physiologic pH, it acts as a permselective membrane to cations, and the passage of counterions across the skin induces an electroosmotic solvent flow that may carry neutral molecules in the anode-to-cathode direction. Components in the solvent flow may be analyzed as described elsewhere herein. In some instances, a reverse iontophoresis apparatus may comprise an anode cell and a cathode cell, each in contact with the skin. The anode cell may be filled, for example, with an aqueous buffer solution (i.e., aqueous Tris buffer) having a pH greater than 4 and an electrolyte (i.e. sodium chloride). The cathode cell can be filled with aqueous buffer. As one example, a first electrode (e.g., an anode) can be inserted into the anode cell and a second electrode (e.g., a cathode) can be inserted in the cathode cell. In some embodiments, the electrodes are not in direct contact with the skin.

A current may be applied to induce reverse iontophoresis, thereby receiving a fluid from the skin. The current applied may be, for example, greater than 0.01 mA, greater than 0.3 mA, greater than 0.1 mA, greater than 0.3 mA, greater than 0.5 mA, or greater than 1 mA. It should be understood that currents outside these ranges may be used as well. The current may be applied for a set period of time. For example, the current may be applied for greater than 30 seconds, greater than 1 minute, greater than 5 minutes, greater than 30 minutes, greater than 1 hour, greater than 2 hours, or greater than 5 hours. It should be understood that times outside these ranges may be used as well.

In one set of embodiments, the device may comprise a substance transfer component in the form of an apparatus for ablating the skin. Without wishing to be bound by any theory, it is believed that ablation comprises removing a microscopic patch of stratum corneum (i.e., ablation forms a micropore), thus allowing access to bodily fluids. In some cases, thermal, radiofrequency, and/or laser energy may be used for ablation. In some instances, thermal ablation may be applied using a heating element. Radiofrequency ablation may be carried out using a frequency and energy capable of heating water and/or tissue. A laser may also be used to irradiate a location on the skin to remove a portion. In some embodiments, the heat may be applied in pulses such that a steep temperature gradient exists essentially perpendicular to the surface of the skin. For example, a temperature of at least 100 °C, at least 200 °C, at least 300 °C, or at least 400 °C may be applied for less than 1 second, less than 0.1 seconds, less than 0.01 seconds, less than 0.005 seconds, or less than 0.001 seconds.

In some embodiments, the device may comprise a substance transfer component in the form of a mechanism for taking a solid sample of tissue. For example, a solid tissue sample may be acquired by methods such as scraping the skin or cutting out a portion. Scraping may comprise a reciprocating action whereby an instrument is scraped along the surface of the skin in two or more directions. Scraping can also be accomplished by a rotating action, for example parallel to the surface of the skin and in one direction (i.e., with a roller drum) or parallel to the surface of the skin and in a circular manner (i.e., with a drilling instrument). A cutting mechanism may comprise a blade capable of making one or more incisions and a mechanism for removing a portion of tissue (i.e., by suction or mechanically picking up) or may use a pincer mechanism for cutting out a portion of tissue. A cutting mechanism may also function by a coring action. For example, a hollow cylindrical device can be penetrated into the skin such that a cylindrical core of tissue may be removed. A solid sample may be analyzed directly or may be liquefied prior to analysis. Liquefaction can comprise treatment with organic solvents, enzymatic solutions, surfactants, etc.

The device may also contain, in some embodiments, a vacuum source. In some cases, the vacuum source is one that is self-contained within the device, i.e., the device need not be connected to an external vacuum source (e.g., a house vacuum) during use of the device to receive blood from the skin. For example, in one set of embodiments, the vacuum source may include a vacuum chamber having a pressure less than atmospheric pressure before blood (or other fluid) is received into the device, i.e., the vacuum chamber is at a "negative pressure" (that is, negative relative to atmospheric pressure) or a "vacuum pressure" (or just having a "vacuum"). For example, the vacuum in the vacuum chamber may be at least about 50 mmHg, at least about 100 mmHg, at least about 150 mmHg, at least about 200 mmHg, at least about 250 mmHg, at least about 300 mmHg, at least about 350 mmHg, at least about 400 mmHg, at least about 450 mmHg, at least about 500 mmHg, at least 550 mmHg, at least 600 mmHg, at least 650 mmHg, at least about 700 mmHg, or at least about 750 mmHg, i.e., below atmospheric pressure. However, in other embodiments, it should be understood that other pressures may be used and/or that different methods may be used to produce other pressures (greater than or less than atmospheric pressure). As non-limiting examples, an external vacuum or a mechanical device may be used as the vacuum source; various additional examples are discussed in detail herein.

As a specific, non-limiting example, in one embodiment, a device may be used to receive fluid without an external power and/or a vacuum source. Examples of such devices include skin patches, strips, tapes, bandages, or the like. For instance, a skin patch may be contacted with the skin of a subject, and a vacuum created through a change in shape of a portion of the skin patch or other device (e.g., using a shape memory polymer), which may be used to deliver and/or receive fluid from the skin. As a specific example, a shape memory polymer may be shaped to be flat at a first temperature (e.g., room temperature) but curved at a second temperature (e.g., body temperature), and when applied to the skin, the shape memory polymer may alter from a flat shape to a curved shape, thereby creating a vacuum. As another example, a mechanical device may be used to create the vacuum, For example, springs, coils, expanding foam (e.g., from a compressed state), a shape memory polymer, shape memory metal, or the like may be stored in a compressed or wound released upon application to a subject, then released (e.g., unwinding, uncompressing, etc.), to mechanically create the vacuum.

Thus, in some cases, the device is "pre-packaged" with a suitable vacuum source (e.g., a pre-evacuated vacuum chamber); for instance, in one embodiment, the device may be applied to the skin and activated in some fashion to create and/or access the vacuum source. In yet another example, a chemical reaction may be used to create a vacuum, e.g., a reaction in which a gas is produced, which can be harnessed to provide the mechanical force to create a vacuum. In still another example, a component of the device may be able to create a vacuum in the absence of mechanical force. In another example, the device may include a self-contained vacuum actuator, for example, chemical reactants, a deformable structure, a spring, a piston, etc.

In one set of embodiments, the device may be able to create a pressure differential (e.g. a vacuum). The pressure differential may be created by a pressure regulator. As used here, "pressure regulator" is a pressure controller component or system able to create a pressure differential between two or more locations. The pressure differential should be at least sufficient to urge the movement of fluid or other material in accordance with various embodiments of the invention as discussed herein, and the absolute pressures at the two or more locations are not important so long as their differential is appropriate, and their absolute values are reasonable for the purposes discussed herein. For example, the pressure regulator may produce a pressure higher than atmospheric pressure in one location, relative to a lower pressure at another location (atmospheric pressure or some other pressure), where the differential between the pressures is sufficient to urge fluid in accordance with the invention. In another example, the regulator or controller will involve a pressure lower than atmospheric pressure (a vacuum) in one location, and a higher pressure at another location(s) (atmospheric pressure or a different pressure) where the differential between the pressures is sufficient to urge fluid in accordance with the invention. Wherever "vacuum" or "pressure" is used herein, in association with a pressure regulator or pressure differential of the invention, it should be understood that the opposite can be implemented as well, as would be understood by those of ordinary skill in the art, i.e., a vacuum chamber can be replaced in many instances with a pressure chamber, for creating a pressure differential suitable for urging the movement of fluid or other material.

The pressure regulator may be an external source of vacuum (e.g. a lab, clinic, hospital, etc., house vacuum line or external vacuum pump), a mechanical device, a vacuum chamber, pre-packaged vacuum chamber, or the like. In some cases, vacuum may be created manually, e.g., by manipulating a syringe pump, a plunger, or the like, or the low pressure may be created mechanically or automatically, e.g., using a piston pump, a syringe, a bulb, a Venturi tube, manual (mouth) suction, etc., or the like. Vacuum chambers can be used in some embodiments, where the device contains, e.g., regions in which a vacuum exits or can be created (e.g. a variable volume chamber, a change in volume of which will affect vacuum or pressure). A vacuum chamber can include pre-evacuated (i.e., pre-packaged) chambers or regions, and/or self-contained actuators.

A "self-contained" vacuum (or pressure) regulator means one that is associated with (e.g., on or within) the device, e.g. one that defines an integral part of the device, or is a separate component constructed and arranged to be specifically connectable to the particular device to form a pressure differential (i.e., not a connection to an external source of vacuum such as a hospital's, clinic's, or lab's house vacuum line, or a vacuum pump suitable for very general use). In some embodiments, the self-contained vacuum source may be actuated in some fashion to create a vacuum within the device. For instance, the self-contained vacuum source may include a piston, a syringe, a mechanical device such as a vacuum pump able to create a vacuum within the device, and/or chemicals or other reactants that can react to increase or decrease pressure which, with the assistance of mechanical or other means driven by the reaction, can form a pressure differential associated with a pressure regulator. Chemical reaction can also drive mechanical actuation with or without a change in pressure based on the chemical reaction itself. A self-contained vacuum source can also include an expandable foam, a shape memory material, or the like.

One category of self-contained vacuum or pressure regulators of the invention includes self-contained assisted regulators. These are regulators that, upon actuation (e.g., the push of a button, or automatic actuation upon, e.g., removal from a package or urging a device against the skin), a vacuum or pressure associated with the device is formed where the force that pressurizes or evacuates a chamber is not the same as the actuation force. Examples of self-contained assisted regulators include chambers evacuated by expansion driven by a spring triggered by actuation, release of a shape-memory material or expandable material upon actuation, initiation of a chemical reaction upon actuation, or the like.

Another category of self-contained vacuum or pressure regulators of the invention are devices that are not necessarily pre-packaged with pressure or vacuum, but which can be pressurized or evacuated, e.g. by a subject, health care professional at a hospital or clinic prior to use, e.g. by connecting a chamber of the device to a source of vacuum or pressure. For example, the subject, or another person, may actuate the device to create a pressure or vacuum within the device, for example, immediately prior to use of the device.

The vacuum or pressure regulator may be a "pre-packaged" pressure or vacuum chamber in the device when used (i.e., the device can be provided ready for use by a subject or practitioner with an evacuated region on or in the device, without the need for any actuation to form the initial vacuum). A pre-packaged pressure or vacuum chamber regulator can, e.g., be a region evacuated (relative to atmospheric pressure) upon manufacture and/or at some point prior to the point at which it is used by a subject or practitioner. For example, a chamber is evacuated upon manufacture, or after manufacture but before delivery of the device to the user, e.g. the clinician or subject. For instance, in some embodiments, the device contains a vacuum chamber having a vacuum of at least about 50 mmHg, at least about 100 mmHg, at least about 150 mmHg, at least about 200 mmHg, at least about 250 mmHg, at least about 300 mmHg, at least about 350 mmHg, at least about 400 mmHg, at least about 450 mmHg, at least about 500 mmHg, at least about 550 mmHg, at least about 600 mmHg, at least about 650 mmHg, at least about 700 mmHg, or at least about 750 mmHg below atmospheric pressure.

In one set of embodiments, a device of the present invention may not have an external power and/or a vacuum source. In some cases, the device is "pre-loaded" with a suitable vacuum source; for instance, in one embodiment, the device may be applied to the skin and activated in some fashion to create and/or access the vacuum source. As one example, a device of the present invention may be contacted with the skin of a subject, and a vacuum created through a change in shape of a portion of the device (e.g., using a shape memory polymer), or the device may contain one or more sealed, self-contained vacuum chambers, where a seal is punctured in some manner to create a vacuum. For instance, upon puncturing the seal, a vacuum chamber may be in fluidic communication with a needle, which can be used to move the skin towards the device, receive fluid from the skin, or the like.

In yet another example, a chemical reaction may be used to create a vacuum, e.g., a reaction in which a gas is produced, which can be harnessed to provide the mechanical force to create a vacuum. In some embodiments, the device may be used to create a vacuum automatically, once activated, without any external control by a user.

In one set of embodiments, the device contains a vacuum chamber that is also used as a storage chamber to receive blood or other fluid received from the subject into the device. For instance, blood received from a subject through or via the substance transfer component may enter the vacuum chamber due to its negative pressure (i.e., because the chamber has an internal pressure less than atmospheric pressure), and optionally stored in the vacuum chamber for later use.

In another set of embodiments, however, the device may include separate vacuum chambers and storage chambers (e.g., chambers to store fluid such as blood from the subject). The vacuum chamber and storage chambers may be in fluid communication, and may have any suitable arrangement. In some embodiments, the vacuum from the vacuum chamber may be used, at least in part, to receive fluid from the skin, which is then directed into a storage chamber, e.g., for later analysis or use, for example, as discussed below. As an example, blood may be received into the device, flowing towards a vacuum chamber, but the fluid may be prevented from entering the vacuum chamber. For instance, in certain embodiments, a material permeable to gas but not to a liquid such as blood may be used. For example, the material may be a membrane such as a hydrophilic or hydrophobic membrane having a suitable porosity, a porous structure, a porous ceramic frit, a dissolvable interface (e.g., formed from a salt or a polymer, etc.), or the like.

In one set of embodiments, the device may include a reservoir able to contain a fluid deliverable to the storage chamber. For example, the reservoir may be in fluid communication a storage chamber within the device via one or more microfluidic channels as discussed herein. The reservoir may contain, for example, a fluid for diluting blood or other fluids within the storage chamber, an anticoagulant, a stabilizing agent, a buffer, a sanitizer, a reaction entity able to react with an analyte suspected of being present in the blood (or other fluid) entering the device, or the like. In some cases, pumps or valves may be used to urge fluid transport from the reservoir to the storage chamber, or in certain embodiments, fluid may be urged into the storage chamber due to differences in pressure, e.g., a vacuum within the storage chamber.

In certain embodiments, the substance transfer component may be fastened on a deployment actuator. In some cases, the deployment actuator can bring the substance transfer component to the skin, and in certain instances, insert the substance transfer component into the skin. For example, the substance transfer component can be moved mechanically, electrically (e.g., with the aid of a servo, which may be computer-controlled), pneumatically, via a piston, a screw, a mechanical linkage, or the like. In one set of embodiments, the deployment actuator can insert the substance transfer component into the skin at a speed of at least about 0.1 cm/s, at least about 0.3 cm/s, about 1 cm/s, at least about 3 cm/s, at least about 10 cm/s, at least about 30 cm/s, at least about 1 m/s, at least about 2 m/s, at least about 3 m/s, at least about 4 m/s, at least about 5 m/s, at least about 6 m/s, at least about 7 m/s, at least about 8 m/s, at least about 9 m/s, at least about 10 m/s, at least about 12 m/s, etc., at the point where the substance transfer component initially contacts the skin. Without wishing to be bound by any theory, it is believed that relatively faster insertion speeds may increase the ability of the substance transfer component to penetrate the skin (without deforming the skin or causing the skin to move in response), and/or decrease the amount of pain felt by the application of the substance transfer component to the skin. Any suitable method of controlling the penetration speed into the skin may be used, include those described herein.

As mentioned, in some embodiments, blood or other bodily fluids may be stored within the device for later use and/or analysis. For example, the device may be attached to a suitable external apparatus able to analyze a portion of the device (e.g., containing the fluid), and/or the external apparatus may remove at least some of the blood or other fluid from the device for subsequent analysis and/or storage. In some cases, however, at least some analysis may be performed by the device itself, e.g., using one or more sensors, etc., contained within the device.

For example, as discussed in detail below, in some cases, a storage chamber may contain a reagent or a reaction entity able to react with an analyte suspected of being present in the blood (or other fluid) entering the device, and in some cases, the reaction entity may be determined to determine the analyte. In some cases, the determination may be made externally of the device, e.g., by determining a color change or a change in fluorescence, etc. The determination may be made by a person, or by an external apparatus able to analyze at least a portion of the device. In some cases, the determination may be made without removing blood from the device, e.g., from the storage chamber. (In other cases, however, blood or other fluid may first be removed from the device before being analyzed.)

In one set of embodiments, the device may include one or more sensors (e.g., ion sensors such as K⁺ sensors, colorimetric sensors, fluorescence sensors, etc.), and/or contain "windows" that allow light to penetrate the device. The windows may be formed of glass, plastic, etc., and may be selected to be at least partially transparent to one or a range of suitable wavelengths, depending on the analyte or condition to be determined. As a specific example, the entire device (or a portion thereof) may be mounted in an external apparatus, and light from the external apparatus may pass through or otherwise interact with at least a portion of the device (e.g., be reflected or refracted via the device) to determine the analyte and/or the reaction entity. For example, an interrogation signal may be passed through a window in the device and used to interrogate at least a portion of the storage chamber, for example, a fluid contained within the storage chamber. For instance, the interrogation signal may be a light signal, a fluorescence signal, an infrared signal, an ultraviolet signal, or the like.

The device, in one aspect, may contain a portion able to determine a fluid or other substance received from the skin. For example, a portion of the device may contain a sensor, or reagents able to interact with an analyte contained or suspected to be present within the received fluid from the subject, for example, a marker for a disease state. The sensor may be embedded within or integrally connected to the device, or positioned remotely but with physical, electrical, and/or optical connection with the device so as to be able to sense a chamber within or fluid from the device. For example, the sensor may be in fluidic communication with fluid received from a subject, directly, via a micro fluidic channel, an analytical chamber, etc. The sensor may be able to sense an analyte, e.g., one that is suspected of being in a fluid received from a subject. For example, a sensor may be free of any physical connection with the device, but may be positioned so as to detect the results of interaction of electromagnetic radiation, such as infrared, ultraviolet, or visible light, which has been directed toward a portion of the device, e.g., a chamber within the device. As another example, a sensor may be positioned on or within the device, and may sense activity in a chamber by being connected optically to the chamber. Sensing communication can also be provided where the chamber is in communication with a sensor fluidly, optically or visually, thermally, pneumatically, electronically, or the like, so as to be able to sense a condition of the chamber. As one example, the sensor may be positioned downstream of a chamber, within a channel such a microfluidic channel, on an external apparatus, or the like.

Thus, the invention provides, in certain embodiments, sensors able to determine an analyte. Such determination may occur within the skin, and/or externally of the subject, e.g., within a device on the surface of the skin, depending on the embodiment. "Determine," in this context, generally refers to the analysis of a species, for example, quantitatively or qualitatively, and/or the detection of the presence or absence of the species. "Determining" may also refer to the analysis of an interaction between two or more species, for example, quantitatively or qualitatively, and/or by detecting the presence or absence of the interaction, e.g. determination of the binding between two species. The species may be, for example, a bodily fluid and/or an analyte suspected of being present in the bodily fluid. "Determining" also means detecting or quantifying interaction between species.

Non-limiting examples of sensors include dye-based detection systems, affinity-based detection systems, microfabricated gravimetric analyzers, CCD cameras, optical detectors, optical microscopy systems, electrical systems, thermocouples and thermistors, pressure sensors, etc. Those of ordinary skill in the art will be able to identify other suitable sensors. The sensor can include a colorimetric detection system in some cases, which may be external to the device, or microfabricated into the device in certain cases. As an example of a colorimetric detection system, if a dye or a fluorescent entity is used (e.g. in a particle), the colorimetric detection system may be able to detect a change or shift in the frequency and/or intensity of the dye or fluorescent entity.

Examples of sensors include, but are not limited to, pH sensors, optical sensors, ion sensors, colorimetric sensors, a sensor able to detect the concentration of a substance, or the like, e.g., as discussed herein. For instance, in one set of embodiments, the device may include an ion selective electrode. The ion selective electrode may be able to determine a specific ion and/or ions such as K⁺, H⁺, Na⁺, Ag⁺, Pb²⁺, Cd²⁺, or the like. Various ion selective electrodes can be obtained commercially. As a non-limiting example, a potassium-selective electrode may include an ion exchange resin membrane, using valinomycin, a potassium channel, as the ion carrier in the membrane to provide potassium specificity.

Examples of analytes that the sensor may be used to determine include, but are not limited to, pH or metal ions, proteins, nucleic acids (e.g. DNA, RNA, etc.), drugs, sugars (e.g., glucose), hormones (e.g., estradiol, estrone, progesterone, progestin, testosterone, androstenedione, etc.), carbohydrates, or other analytes of interest. Other conditions that can be determined can include pH changes, which may indicate disease, yeast infection, periodontal disease at a mucosal surface, oxygen or carbon monoxide levels which indicate lung dysfunction, and drug levels, e.g., legal prescription levels of drugs such as coumadin, other drugs such as nicotine, or illegal such as cocaine. Further examples of analytes include those indicative of disease, such as cancer specific markers such as CEA and PSA, viral and bacterial antigens, and autoimmune indicators such as antibodies to double stranded DNA, indicative of Lupus. Still other conditions include exposure to elevated carbon monoxide, which could be from an external source or due to sleep apnea, too much heat (important in the case of babies whose internal temperature controls are not fully self-regulating) or from fever. Still other potentially suitable analytes include various pathogens such as bacteria or viruses, and/or markers produced by such pathogens.

As additional non-limiting examples, the sensor may contain an antibody able to interact with a marker for a disease state, an enzyme such as glucose oxidase or glucose 1-dehydrogenase able to detect glucose, or the like. The analyte may be determined quantitatively or qualitatively, and/or the presence or absence of the analyte within the received fluid may be determined in some cases. Those of ordinary skill in the art will be aware of many suitable commercially-available sensors, and the specific sensor used may depend on the particular analyte being sensed. For instance, various non-limiting examples of sensor techniques include pressure or temperature measurements, spectroscopy such as infrared, absorption, fluorescence, UV/visible, FTIR ("Fourier Transform Infrared Spectroscopy"), or Raman; piezoelectric measurements; immunoassays; electrical measurements, electrochemical measurements (e.g., ion-specific electrodes); magnetic measurements, optical measurements such as optical density measurements; circular dichroism; light scattering measurements such as quasielectric light scattering; polarimetry; refractometry; chemical indicators such as dyes; or turbidity measurements, including nephelometry.

In one set of embodiments, a sensor in the device may be used to determine a condition of the blood present within the device. For example, the sensor may indicate the condition of analytes commonly found within the blood, for example, O₂, K⁺, hemoglobin, Na⁺, glucose, or the like. As a specific non-limiting example, in some embodiments, the sensor may determine the degree of hemolysis within blood contained within the device. Without wishing to be bound by any theory, it is believed that in some cases, hemolysis of red blood cells may cause the release of potassium ions and/or free hemoglobin into the blood. By determining the levels of potassium ions, and/or hemoglobin (e.g., by subjecting the device and/or the blood to separate cells from plasma, then determining hemoglobin in the plasma using a suitable colorimetric assay), the amount of blood lysis or "stress" experienced by the blood contained within the device may be determined. Accordingly, in one set of embodiments, the device may indicate the usability of the blood (or other fluid) contained within the device, e.g., by indicating the degree of stress or the amount of blood lysis. Other examples of devices suitable for indicating the usability of the blood (or other fluid) contained within the device are also discussed herein (e.g., by indicating the amount of time blood has been contained in the device, the temperature history of the device, etc.).

For instance, fluids received from the subject will often contain various analytes within the body that are important for diagnostic purposes, for example, markers for various disease states, such as glucose (e.g., for diabetics); other example analytes include ions such as sodium, potassium, chloride, calcium, magnesium, and/or bicarbonate (e.g., to determine dehydration); gases such as carbon dioxide or oxygen; H⁺ (i.e., pH); metabolites such as urea, blood urea nitrogen or creatinine; hormones such as estradiol, estrone, progesterone, progestin, testosterone, androstenedione, etc. (e.g., to determine pregnancy, illicit drug use, or the like); or cholesterol. Other examples include insulin, or hormone levels. As discussed herein, certain embodiments of the present invention are generally directed at methods for receiving fluids from the body, and optionally determining one or more analytes within the received fluid. Thus, in some embodiments, at least a portion of the fluid may be stored, and/or analyzed to determine one or more analytes, e.g., a marker for a disease state, or the like. The fluid received from the skin may be subjected to such uses, and/or one or more materials previously delivered to the skin may be subject to such uses.

Still other potentially suitable analytes include various pathogens such as bacteria or viruses, and/or markers produced by such pathogens. Thus, in certain embodiments of the invention, as discussed below, one or more analytes within the pooled region of fluid may be determined in some fashion, which may be useful in determining a past, present and/or future condition of the subject.

In one set of embodiments, the analytes may interact with one or more reaction entities, e.g., able to interact with an analyte suspected of being present in blood or other fluid received from a subject. In some cases, the reaction entities may be immobilized on a substrate, for example, on a "test strip" or other substrate containing one or more reaction entities. The test strip may be, for example, paper, plastic, metal, or the like, and may be useable only once or multiple times, depending on the test strip. The test strip may have any size or shape suitable for insertion or application to the device. The test strip, in some cases, may be obtained commercially. For example, test strips are commercially available that can be used for detection of glucose, pH, components in urine, or the like. In some cases, the device may be constructed and arranged such that the test strip is removable from the device. For example, a test strip (e.g., a commercially-available test strip or a custom-made test strip) may be inserted into a device, then the device may receive a sample of fluid from the subject such that the fluid interacts with one or more reaction entities on the test strip. In some cases, the device may also read the test strip, e.g., by determining a color change, a chemical reaction, or the like, and/or the test strip may be removed from the device and interfaced with external equipment, for example, able to analyze a portion of the test strip to determine an analyte (for example, as is discussed herein).

In one aspect, the device may be interfaced with an external apparatus able to determine an analyte contained within a fluid in the device, for example within a storage chamber as discussed herein. For example, the device may be mounted on an external holder, the device may include a port for transporting fluid out of the device, the device may include a window for interrogating a fluid contained within the device, or the like. Examples may be seen in U.S. Patent Application Serial No. 13/006,165 filed on January 13, 2011, entitled "Sampling Device Interfaces," incorporated herein by reference in its entirety.

In some embodiments, the device may connected to an external apparatus for determining at least a portion of the device, a fluid removed from the device, an analyte suspected of being present within the fluid, or the like. For example, the device may be connected to an external analytical apparatus, and fluid removed from the device for later analysis, or the fluid may be analyzed within the device *in situ,* e.g., by adding one or more reaction entities to the device, for instance, to a storage chamber, or to analytical chamber within the device. For example, in one embodiment, the external apparatus may have a port or other suitable surface for mating with a port or other suitable surface on the device, and blood or other fluid can be removed from the device using any suitable technique, e.g., using vacuum or pressure, etc. The blood may be removed by the external apparatus, and optionally, stored and/or analyzed in some fashion. For example, in one set of embodiments, the device may include an exit port for removing a fluid from the device (e.g., blood). In some embodiments, fluid contained within a storage chamber in the device may be removed from the device, and stored for later use or analyzed outside of the device. In some cases, the exit port may be separate from the substance transfer component.

In one set of embodiments, the device may include an anticoagulant or a stabilizing agent for stabilizing the fluid received from the skin. For example, the fluid may be stored within the device for a certain period of time, and/or the device (or a portion thereof) may be moved or shipped to another location for analysis or later use. For instance, a device may contain anticoagulant or a stabilizing agent in a storage chamber. In some cases, more than one anticoagulant may be used, e.g., in the same storage chamber, or in more than one storage chamber.

As a specific non-limiting example, an anticoagulant may be used for blood received from the skin. Examples of anticoagulants include, but are not limited to, heparin, citrate, thrombin, oxalate, ethylenediaminetetraacetic acid (EDTA), sodium polyanethol sulfonate, acid citrate dextrose. Other agents may be used in conjunction or instead of anticoagulants, for example, stabilizing agents such as solvents, diluents, buffers, chelating agents, antioxidants, binding agents, preservatives, antimicrobials, or the like. Examples of preservatives include, for example, benzalkonium chloride, chlorobutanol, parabens, or thimerosal. Non-limiting examples of antioxidants include ascorbic acid, glutathione, lipoic acid, uric acid, carotenes, alpha-tocopherol, ubiquinol, or enzymes such as catalase, superoxide dismutase, or peroxidases. Examples of microbials include, but are not limited to, ethanol or isopropyl alcohol, azides, or the like. Examples of chelating agents include, but are not limited to, ethylene glycol tetraacetic acid or ethylenediaminetetraacetic acid. Examples of buffers include phosphate buffers such as those known to ordinary skill in the art.

As mentioned, in one set of embodiments, a device of the invention as discussed herein may be shipped to another location for analysis. In some cases, the device may include an anticoagulant or a stabilizing agent contained within the device, e.g., within a storage chamber for the fluid. Thus, for example, fluid such as blood received from the skin may be delivered to a chamber (e.g., a storage chamber) within the device, then the device, or a portion of the device (e.g., a module) may be shipped to another location for analysis. Any form of shipping may be used, e.g., via mail.

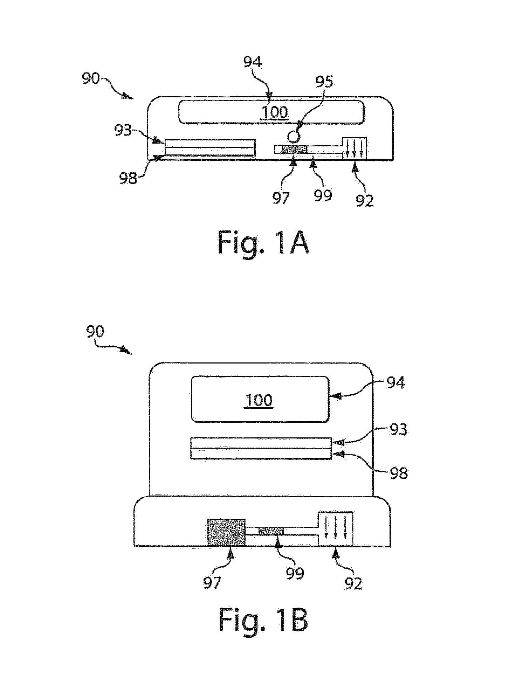

Non-limiting examples of various devices of the invention are shown in Fig. 1. In Fig. 1A, device 90 is used for receiving a fluid from a subject when the device is placed on the skin of a subject. Device 90 includes sensor 95 and substance transfer component 92, e.g., including a needle, a microneedle, etc., as discussed herein. In fluidic communication with substance transfer component 92 via fluidic channel 99 is sensing chamber 97. In one embodiment, sensing chamber 97 may contain agents such as particles, enzymes, dyes, etc., for analyzing bodily fluids, such as interstitial fluid or blood. In some cases, fluid may be received using substance transfer component 92 by a vacuum, for example, a self-contained vacuum contained within device 90. Optionally, device 90 also contains a display 94 and associated electronics 93, batteries or other power supplies, etc., which may be used to display sensor readings obtained via sensor 95. In addition, device 90 may also optionally contain memory 98, transmitters for transmitting a signal indicative of sensor 95 to a receiver, etc.

In the example shown in Fig. 1A, device 90 may contain a vacuum source (not shown) that is self-contained within device 90, although in other embodiments, the vacuum source may be external to device 90. (In still other instances, other systems may be used to deliver and/or receive fluid from the skin, as is discussed herein.) In one embodiment, after being placed on the skin of a subject, the skin may be drawn upward into a recess of the substance transfer component 92, for example, upon exposure to the vacuum source. Access to the vacuum source may be controlled by any suitable method, e.g., by piercing a seal or a septum; by opening a valve or moving a gate, etc. For instance, upon activation of device 90, e.g., by the subject, remotely, automatically, etc., the vacuum source may be put into fluidic communication with the recess such that skin is drawn into the recess due to the vacuum. Skin drawn into the recess may come into contact with a skin insertion object (e.g., solid or hollow needles), which may, in some cases, pierce the skin and allow a fluid to be delivered and/or received from the skin. In another embodiment, a skin insertion object may be actuated and moved downward to come into contact with the skin, and optionally retracted after use.

Another non-limiting example of a device is shown in Fig. 1B. This figure illustrates a device useful for delivering a fluid to the subject. Device 90 in this figure includes substance transfer component 92, e.g., including a needle, a microneedle, etc., as discussed herein. In fluidic communication with substance transfer component 92 via fluidic channel 99 is chamber 97, which may contain a drug or other agent to be delivered to the subject. In some cases, fluid may be delivered with a pressure controller, and/or received using substance transfer component 92 by a vacuum, for example, a self-contained vacuum contained within device 90. For instance, upon creating a vacuum, skin may be drawn up towards substance transfer component 92, and the substance transfer component 92 may pierce the skin. Fluid from chamber 97 can then be delivered into the skin through fluid channel 99 and substance transfer component 92. Optionally, device 90 also contains a display 94 and associated electronics 93, batteries or other power supplies, etc., which may be used control delivery of fluid to the skin. In addition, device 90 may also optionally contain memory 98, transmitters for transmitting a signal indicative of device 90 or fluid delivery to a receiver, etc.

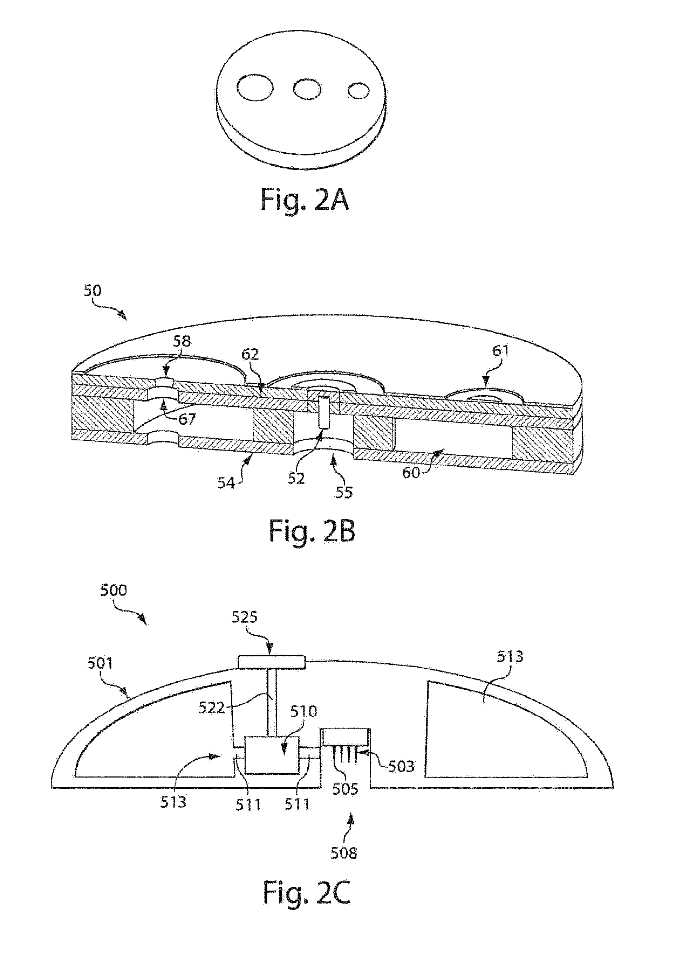

Yet another non-limiting example of a device of the invention is shown in Fig. 2. Fig. 2A illustrates a view of the device (with the cover removed), while Fig. 2B schematically illustrates the device in cross-section. In Fig. 2B, device 50 includes a needle 52 contained within a recess 55. Needle 52 may be solid or hollow, depending on the embodiment. Device 50 also includes a self-contained vacuum chamber 60, which wraps around the central portion of the device where needle 52 and recess 55 are located. A channel 62 connects vacuum chamber 60 with recess 55, separated by a foil or a membrane 67. Also shown in device 50 is button 58. When pushed, button 58 breaks foil 67, thereby connecting vacuum chamber 50 with recess 55, creating a vacuum in recess 55. The vacuum may be used, for example, to draw skin into recess 55, preferably such that it contacts needle 52 and pierces the surface, thereby gaining access to an internal fluid. The fluid may be controlled, for example, by controlling the size of needle 52, and thereby the depth of penetration. For example, the penetration may be limited to the epidermis, e.g., to collect interstitial fluid, or to the dermis, e.g., to collect blood. In some cases, the vacuum may also be used to at least partially secure device 50 on the surface of the skin, and/or to assist in the receiving of fluid from the skin. For instance, fluid may flow into channel 62 under action of the vacuum, and optionally to sensor 61, e.g., for detection of an analyte contained within the fluid. For instance, sensor 61 may produce a color change if an analyte is present, or otherwise produce a detectable signal.

Other components may be added to the example of the device illustrated in Fig. 2, in some embodiments of the invention. For example, device 50 may contain a cover, displays, ports, transmitters, sensors, channels such as microfluidic channels, chambers, and/or various electronics, e.g., to control or monitor fluid transport into or out of device 50, to determine an analyte present within a fluid delivered and/or received from the skin, to determine the status of the device, to report or transmit information regarding the device and/or analytes, or the like, as is discussed in more detail herein. As another example, device 50 may contain an adhesive, e.g., on surface 54, for adhesion of the device to the skin.

Yet another non-limiting example is illustrated with reference to Fig. 2C. In this example, device 500 includes a housing 501, and an associated substance transfer component 503. Substance transfer component 503 includes a plurality of needles or microneedles 505, although other skin insertion objects or flow activators as discussed herein may also be used. Also shown in Fig. 2C is sensor 510, connected via channels 511 to recess 508 containing needles or microneedles 505. Chamber 513 may be a self-contained vacuum chamber, and chamber 513 may be in fluidic communication with recess 508 via channel 511, for example, as controlled by a controller or an actuator (not shown). In this figure, device 500 also contains display 525, which is connected to sensor 510 via electrical connection 522. As an example of use of device 500, when fluid is drawn from the skin (e.g., blood, interstitial fluid, etc.), the fluid may flow through channel 511 to be determined by sensor 510, e.g., due to action of the vacuum from vacuum chamber 513. In some cases, the vacuum is used, for example, to draw skin into recess 508, preferably such that it contacts needles or microneedles 505 and pierces the surface of the skin to gain access to a fluid internal of the subject, such as blood or interstitial fluid, etc. The fluid may be controlled, for example, by controlling the size of needle 505, and thereby the depth of penetration. For example, the penetration may be limited to the epidermis, e.g., to collect interstitial fluid, or to the dermis, e.g., to collect blood. Upon determination of the fluid and/or an analyte present or suspected to be present within the fluid, a microprocessor or other controller may display on display 525 a suitable signal. As is discussed below, a display is shown in this figure by way of example only; in other embodiments, no display may be present, or other signals may be used, for example, lights, smell, sound, feel, taste, or the like.

In some cases, more than one substance transfer component may be present within the device. For instance, the device may be able to be used repeatedly, and/or the device may be able to deliver and/or receive fluid at more than one location on a subject, e.g., sequentially and/or simultaneously. In some cases, the device may be able to simultaneously deliver and receive fluid to and from a subject. Each of these substance transfer components may independently have the same or different structures, depending on the particular application, and they may have structures such as those described herein.

In one set of embodiments, the device is able to shield the needles or microneedles (or other substance transfer component) against accidental contact or insertion, e.g., when the device is not actively being used. For instance, the device may be constructed and arranged to prevent "needlesticks" or other "sharps" contacts when the device is not used. For instance, in certain embodiments, an array of needles may be actuated to move towards the skin when the device is placed on the skin, e.g., mechanically, electrically (e.g., with the aid of a servo, which may be computer-controlled), pneumatically, etc. An example may be seen in Fig. 18. In some cases, the array of needles may also be actuated away from the skin when the device is not in use. Thus, during use, the needles are brought into contact with the skin, but before or after use, the needles are positioned within the device such that the user of the device cannot accidently come into contact with the needles.

In another set of embodiments, a shield or other cover able to cover some or all of the needles (or other substance transfer component) may be used to cover the needles when the device is not in use. For example, the device may have a cover or a shield that is moved over the needles (e.g., over a shaft or alcove for the needles), such that no accidental contact with the needles can occur. The shield may be formed out of any suitable material, e.g., glass, plastic, metal, rubber, etc. In one set of embodiments, the shield covers the needles and is away from the needles when the device is to be used. For instance, the shield may be moved mechanically, electrically (e.g., with the aid of a servo, which may be computer-controlled), pneumatically, spring-loaded, etc. In another set of embodiments, the shield does not move, but the needles (or other substance transfer component) may be pushed through the shield in order to reach the skin of the subject. The shield may take any suitable form, for example, a relatively flat layer, a "cap" or a domed structure that covers the needles, or the like.

In yet another set of embodiments, one or more needles or other substance transfer components may be withdrawn into a sleeve or other surrounding shield when not in use. For example, the sleeve may be formed out of any suitable material that prevents accidental contact with the substance transfer component from occurring when the device is not in use. Non-limiting examples of such materials include glass, plastic, metal, rubber, etc. The sleeve may surround (or at least partially surround) all of the needles, or a plurality of sleeves may surround each of the needles, etc. The sleeve may be extended and/or retracted to surround the needles when not in use, and/or the needles may be extended and/or retracted in and out of the sleeve during use. In some cases, the sleeve may be formed out of a rigid material that cannot be readily deformed via casual contact with a person.

One set of embodiments of the invention is generally directed to a device where the activation of a device for delivering and/or receiving fluid from the skin of a subject, and the actual act of delivering and/or receiving fluid, are not essentially simultaneously. Thus, time may elapse between activation and the actual delivery and/or receiving, wherein the subject's attention may be diverted elsewhere, e.g., simply by everyday occurrences, or due to boredom in the interim. The subject may, in some cases, be free to move on to do other things, e.g., while wearing the device, for example, if the device is wearable or portable. For example, the time period can be at least about 1 second, at least about 2 seconds, at least about 3 seconds, at least about 5 seconds, at least about 8 seconds, at least about 10 seconds, at least about 15 seconds, at least about 20 seconds, 30 seconds, at least about 45 seconds, at least about 1 minute, at least about 2 minutes, at least 3 minutes, at least about 4 minutes, at least about 5 minutes, at least about 10 minutes, at least about 15 minutes, at least about 30 minutes, at least about 45 minutes, at least about 1 hour, etc. In some cases, the time period can be randomly determined, e.g., by the device, further decreasing the subject's expectation of the actual fluid delivery and/or receiving. In some cases, the time may be sufficient that a subject may have forgotten about the device. Thus, due to the passage of time between the time the device is initially activated, and the time the device begins to deliver and/or receive fluid (or other substances), the subject may no longer be expecting or sure of the delivery and/or reception of fluid, and thus, the subject may perceive less associated pain.

As another example, in one set of embodiments, the device may be activated at a specific time of day, e.g., at 3 AM (or other time, for example, when the subject is asleep). For instance, the device may be automatically activated, without any external intervention. In another set of embodiments, the device may be activated after a specific activity. For example, the device may be activated after the subject eats, exercises, or goes to sleep. Thus, for example, the device may deliver and/or receive fluid (or other substances) to and/or from the skin of the subject while the subject is asleep, thereby reducing (or eliminating) the subject's expectation of pain associated with the device. The device may include a timed activator (for example, a computer-controlled activator that is automatically activated when the computer registers a particular time), and/or an activator connected to one or more sensors, for example, a kinetic sensor able to detect movement or lack thereof, e.g., rapid movement (e.g., during exercise) or little movement (e.g., during sleep).

In some embodiments, the device may be an electrical and/or a mechanical device applicable or affixable to the surface of the skin, e.g., using adhesive, or other techniques such as those described herein. The adhesive may be permanent or temporary, and may be used to affix the device to the surface of the skin. The adhesive may be any suitable adhesive, for example, a pressure sensitive adhesive, a contact adhesive, a permanent adhesive, a hydrogel, a cyanoacrylate, a glue, a gum, hot melts, an epoxy, or the like. In some cases, the adhesive is chosen to be biocompatible or hypoallergenic.

In another set of embodiments, the device may be mechanically held to the skin, for example, the device may include mechanical elements such as straps, belts, buckles, strings, ties, elastic bands, or the like. For example, a strap may be worn around the device to hold the device in place against the skin of the subject. In yet another set of embodiments, a combination of these and/or other techniques may be used. As one non-limiting example, the device may be affixed to a subject's arm or leg using adhesive and a strap.

As used herein, the term "fluid" generally refers to a substance that tends to flow and to conform to the outline of its container. Typically, fluids are materials that are unable to withstand a static shear stress, and when a shear stress is applied, the fluid experiences a continuing and permanent distortion. The fluid may have any suitable viscosity that permits at least some flow of the fluid. Non-limiting examples of fluids include liquids and gases, but may also include free-flowing solid particles, viscoelastic fluids, and the like. For example, the fluid may include a flowable matrix or a gel, e.g., formed from biodegradable and/or biocompatible material such as polylactic acid, polyglycolic acid, poly(lactic-co-glycolic acid), etc., or other similar materials.

According to one aspect of the invention, the device is of a relatively small size. In some embodiments, the device may be sized such that it is wearable and/or carryable by a subject. For example, the device may be self-contained, needing no wires, cables, tubes, external structural elements, or other external support. The device may be positioned on any suitable position of the subject, for example, on the arm or leg, on the back, on the abdomen, etc. As mentioned, in some embodiments, the device may be affixed or held onto the surface of the skin using any suitable technique, e.g., using adhesives, mechanical elements such as straps, belts, buckles, strings, ties, elastic bands, or the like. In some cases, the device may be positioned on the subject such that the subject is able to move around (e.g., walking, exercising, typing, writing, drinking or eating, using the bathroom, etc.) while wearing the device.

In some embodiments, the device is relatively lightweight. For example, the device may have a mass of no more than about 1 kg, no more than about 300 g, no more than about 150 g, no more than about 100 g, no more than about 50 g, no more than about 30 g, no more than about 25 g, no more than about 20 g, no more than about 10 g, no more than about 5 g, or no more than about 2 g. For instance, in various embodiments, the device has a mass of between about 2 g and about 25 g, a mass of between about 2 g and about 10 g, a mass of between 10 g and about 50 g, a mass of between about 30 g and about 150 g, etc.

The device, in some cases, may be relatively small. For example, the device may be constructed and arranged to lie relatively close to the skin. Thus, for instance, the device may have a largest vertical dimension, extending from the skin of the subject when the device is positioned on the skin, of no more than about 25 cm, no more than about 10 cm, no more than about 7 cm, no more than about 5 cm, no more than about 3 cm, no more than about 2 cm, no more than about 1 cm, no more than about 8 mm, no more than about 5 mm, no more than about 3 mm, no more than about 2 mm, no more than about 1 mm, or no more than about 0.5 mm. In some cases, the device may have a largest vertical dimension of between about 0.5 cm and about 1 cm, between about 2 and about 3 cm, between about 2.5 cm and about 5 cm, between about 2 cm and about 7 cm, between about 0.5 mm and about 7 cm, etc.

In another set of embodiments, the device may have a relatively small size. For example, the device may have a largest lateral dimension (e.g., parallel to the skin) of no more than about 25 cm, no more than about 10 cm, no more than about 7 cm, no more than about 5 cm, no more than about 3 cm, no more than about 2 cm, or no more than about 1 cm. In some cases, the device may have a largest lateral dimension of between about 0.5 cm and about 1 cm, between about 2 and about 3 cm, between about 2.5 cm and about 5 cm, between about 2 cm and about 7 cm, etc.

Combinations of these and/or other dimensions are also possible in other embodiments. As non-limiting examples, the device may have a largest lateral dimension of no more than about 5 cm, a largest vertical dimension of no more than about 1 cm, and a mass of no more than about 25 g; or the device may have a largest lateral dimension of no more than about 5 cm, a largest vertical dimension of no more than about 1 cm, and a mass of no more than about 25 g; etc.

In certain embodiments, the may also include a device actuator. The device actuator may be constructed and arranged to cause exposure of the substance transfer component to the skin upon actuation of the device actuator. For example, the activator may cause the substance transfer component to release a chemical to contact the skin, a microneedle or other substance transfer component to be driven into the skin, a vacuum to be applied to the skin, a jet of fluid to be directed to the skin, or the like. The device actuator may be actuated by the subject, and/or by another person (e.g., a health care provider), or the device itself may be self-actuating, e.g., upon application to the skin of a subject. The actuator may be actuated once, or multiple times in some cases.

Thus, in one aspect, the device may be constructed and arranged to be activated remotely. For example, the device may be activated upon interaction with another entity, for example, another device, or a target. As one example, the device may not be activatable until it receives or detects a target or a signal, e.g., from another device. As another example, the device may record the target or signal, or otherwise associate the target or signal with fluid received from the subject. For example, a device may record, e.g., in memory, data about an event where fluid was received from the subject, for instance, along with time data. The device may be activated simply by proximity to the target or signal (e.g., indicating that the subject from which fluid is delivered to and/or received from is the correct subject), or the device may be activated or remotely activated upon a certain event. For instance, the device may be remotely activated, for example, by a doctor or other medical personnel, or the device may be activated when the subject does something, e.g., eats, exercises, or goes to sleep. In one set of embodiments, the device includes a scanner able to scan a signal, for example, an optical signal, a sonic signal (including ultrasound), a radio signal, an ultraviolet signal, or the like. The scanner may be any device that can detect a signal, such as a signal external to the device. For example, the scanner may comprise an antenna such as a radio antenna, a photoelectrode, a photodiode, a bar code scanner, an ultraviolet sensor, an infrared sensor, a microphone, a receiver for detection of radio, sound, light, or the like. Many such scanners are available commercially.

As a specific example, in one set of embodiments, the device may contain a receiver and the subject may contain a transmitter that the receiver detects signals from. For example, the transmitter may be radio waves, sound waves, light waves, or the like. In one set of embodiments, the subject possesses an RFID transmitter or "tag" and the device may contain a receiver able to detect signals from the RFID tag. The tag may be positioned within any suitable apparatus. For instance, the tag may be embedded within a card or a paper to be carried by the subject, within a strap, belt, elastic band, wristband, etc., to be worn by the subject, or in some cases, the RFID tag may be implanted into the subject. In one set of embodiments, the tag is attached to the subject, either temporarily or permanently. The device can detect the presence of the nearby RFID tags, which assures that the device is being worn or used by the correct subject.

As another example, the subject may possess a scannable target, for example, a bar code, which may be a 1- or 2-dimensional barcode, and may code information based on lines, colors, patterns, shapes, or any other features or combinations of features. The bar code or other scannable target may be positioned within any suitable apparatus. For instance, the target may be embedded within a card to be carried by the subject, within a strap, belt, elastic band, wristband, etc. In one set of embodiments, the target is attached to the subject, either temporarily or permanently. In some cases, the target may be implanted into the subject (e.g., as in a tattoo, particles embedded within the skin as is described herein, etc.). The implantation can be either temporarily or permanently. The device may contain a scanner able to scan the scannable target. Prior to or during use, the device may be held next to the scannable target such that the device is able to scan the scannable target in order to verify that the subject is the correct subject, for example, using a scanner contained within the device. For instance, the subject may possess a barcode strapped to the wrist or ankle, and the device passed over the barcode in order to activate it, e.g., by using a bar code scanner contained within the device.

In one set of embodiments, the device may include channels such as microfluidic channels, which may be used to deliver and/or receive fluids and/or other materials into or out of the skin. In some cases, the microfluidic channels are in fluid communication with a substance transfer component that is used to deliver and/or receive fluids to or from the skin. For example, in one set of embodiments, the device may include a hypodermic needle that can be inserted into the skin, and fluid may be delivered into the skin via the needle and/or received from the skin via the needle. The device may also include one or more microfluidic channels to contain fluid for delivery to the needle, e.g., from a source of fluid, and/or to receive fluid from the skin, e.g., for delivery to an analytical chamber within the device, to a reservoir for later analysis, or the like.

In some cases, more than one chamber may be present within the device, and in some cases, some or all of the chambers may be in fluidic communication, e.g., via channels such as microfluidic channels. In various embodiments, a variety of chambers and/or channels may be present within the device, depending on the application. For example, the device may contain chambers for sensing an analyte, chambers for holding reagents, chambers for controlling temperature, chambers for controlling pH or other conditions, chambers for creating or buffering pressure or vacuum, chambers for controlling or dampening fluid flow, mixing chambers, or the like.

Thus, in one set of embodiments, the device may include a microfluidic channel. As used herein, "microfluidic," "microscopic," "microscale," the "micro-" prefix (for example, as in "microchannel"), and the like generally refers to elements or articles having widths or diameters of less than about 1 mm, and less than about 100 microns (micrometers) in some cases. In some embodiments, larger channels may be used instead of, or in conjunction with, microfluidic channels for any of the embodiments discussed herein. For example, channels having widths or diameters of less than about 10 mm, less than about 9 mm, less than about 8 mm, less than about 7 mm, less than about 6 mm, less than about 5 mm, less than about 4 mm, less than about 3 mm, or less than about 2 mm may be used in certain instances. In some cases, the element or article includes a channel through which a fluid can flow. In all embodiments, specified widths can be a smallest width (i.e. a width as specified where, at that location, the article can have a larger width in a different dimension), or a largest width (i.e. where, at that location, the article has a width that is no wider than as specified, but can have a length that is greater). Thus, for instance, the microfluidic channel may have an average cross-sectional dimension (e.g., perpendicular to the direction of flow of fluid in the microfluidic channel) of less than about 1 mm, less than about 500 microns, less than about 300 microns, or less than about 100 microns. In some cases, the microfluidic channel may have an average diameter of less than about 60 microns, less than about 50 microns, less than about 40 microns, less than about 30 microns, less than about 25 microns, less than about 10 microns, less than about 5 microns, less than about 3 microns, or less than about 1 micron.

A "channel," as used herein, means a feature on or in an article (e.g., a substrate) that at least partially directs the flow of a fluid. In some cases, the channel may be formed, at least in part, by a single component, e.g. an etched substrate or molded unit. The channel can have any cross-sectional shape, for example, circular, oval, triangular, irregular, square or rectangular (having any aspect ratio), or the like, and can be covered or uncovered (i.e., open to the external environment surrounding the channel). In embodiments where the channel is completely covered, at least one portion of the channel can have a cross-section that is completely enclosed, and/or the entire channel may be completely enclosed along its entire length with the exception of its inlet and outlet.

A channel may have any aspect ratio, e.g., an aspect ratio (length to average cross-sectional dimension) of at least about 2:1, more typically at least about 3:1, at least about 5:1, at least about 10:1, etc. As used herein, a "cross-sectional dimension," in reference to a fluidic or microfluidic channel, is measured in a direction generally perpendicular to fluid flow within the channel. A channel generally will include characteristics that facilitate control over fluid transport, e.g., structural characteristics and/or physical or chemical characteristics (hydrophobicity vs. hydrophilicity) and/or other characteristics that can exert a force (e.g., a containing force) on a fluid. The fluid within the channel may partially or completely fill the channel. In some cases the fluid may be held or confined within the channel or a portion of the channel in some fashion, for example, using surface tension (e.g., such that the fluid is held within the channel within a meniscus, such as a concave or convex meniscus). In an article or substrate, some (or all) of the channels may be of a particular size or less, for example, having a largest dimension perpendicular to fluid flow of less than about 5 mm, less than about 2 mm, less than about 1 mm, less than about 500 microns, less than about 200 microns, less than about 100 microns, less than about 60 microns, less than about 50 microns, less than about 40 microns, less than about 30 microns, less than about 25 microns, less than about 10 microns, less than about 3 microns, less than about 1 micron, less than about 300 nm, less than about 100 nm, less than about 30 nm, or less than about 10 nm or less in some cases. In one embodiment, the channel is a capillary.

After receipt of the fluid into the device, the device, or a portion thereof, may be removed from the skin of the subject, e.g., by the subject or by another person. For example, the entire device may be removed, or a portion of the device containing the storage reservoir may be removed from the device, and optionally replaced with another storage reservoir. Thus, for instance, in one embodiment, the device may contain two or more modules, for example, a first module that is able to cause receiving of fluid from the skin into a storage reservoir, and a second module containing the storage module. In some cases, the module containing the storage reservoir may be removed from the device. Other examples of modules and modular systems are discussed below; other examples are discussed in U.S. Provisional Patent Application Serial No. 61/256,931, filed October 30, 2009, entitled "Modular Systems for Application to the Skin," incorporated by reference herein in its entirety.

The received fluid may then be sent to a clinical and/or laboratory setting, e.g., for analysis. In some embodiments, the entire device may be sent to the clinical and/or laboratory setting; in other embodiments, however, only a portion of the device (e.g., a module containing a storage reservoir containing the fluid) may be sent to the clinical and/or laboratory setting. In some cases, the fluid may be shipped using any suitable technique (e.g., by mail, by hand, etc.). In certain instances, the subject may give the fluid to appropriate personnel at a clinical visit. For instance, a doctor may prescribe a device as discussed above for use by the subject, and at the next doctor visit, the subject may give the doctor the received fluid, e.g., contained within a device or module.

A variety of materials and methods, according to certain aspects of the invention, can be used to form the device, e.g., microfluidic channels, chambers, etc. For example, various components of the invention can be formed from solid materials, in which the channels can be formed via micromachining, film deposition processes such as spin coating and chemical vapor deposition, laser fabrication, photolithographic techniques, etching methods including wet chemical or plasma processes, and the like. See, for example, Scientific American, 248:44-55, 1983 (Angell, *et al*).

In one set of embodiments, various components of the systems and devices of the invention can be formed of a polymer, for example, an elastomeric polymer such as polydimethylsiloxane ("PDMS"), polytetrafluoroethylene ("PTFE" or Teflon^{®}), or the like. For instance, according to one embodiment, a microfluidic channel may be implemented by fabricating the fluidic system separately using PDMS or other soft lithography techniques (details of soft lithography techniques suitable for this embodiment are discussed in the references entitled "Soft Lithography," by Younan Xia and George M. Whitesides, published in the Annual Review of Material Science, 1998, Vol. 28, pages 153-184, and "Soft Lithography in Biology and Biochemistry," by George M. Whitesides, Emanuele Ostuni, Shuichi Takayama, Xingyu Jiang and Donald E. Ingber, published in the Annual Review of Biomedical Engineering, 2001, Vol. 3, pages 335-373; each of these references is incorporated herein by reference).

Other examples of potentially suitable polymers include, but are not limited to, polyethylene terephthalate (PET), polyacrylate, polymethacrylate, polycarbonate, polystyrene, polyethylene, polypropylene, polyvinylchloride, cyclic olefin copolymer (COC), polytetrafluoroethylene, a fluorinated polymer, a silicone such as polydimethylsiloxane, polyvinylidene chloride, bis-benzocyclobutene ("BCB"), a polyimide, a fluorinated derivative of a polyimide, or the like. Combinations, copolymers, or blends involving polymers including those described above are also envisioned. The device may also be formed from composite materials, for example, a composite of a polymer and a semiconductor material.

In some embodiments, various components of the invention are fabricated from polymeric and/or flexible and/or elastomeric materials, and can be conveniently formed of a hardenable fluid, facilitating fabrication via molding (e.g. replica molding, injection molding, cast molding, etc.). The hardenable fluid can be essentially any fluid that can be induced to solidify, or that spontaneously solidifies, into a solid capable of containing and/or transporting fluids contemplated for use in and with the fluidic network. In one embodiment, the hardenable fluid comprises a polymeric liquid or a liquid polymeric precursor (i.e. a "prepolymer"). Suitable polymeric liquids can include, for example, thermoplastic polymers, thermoset polymers, waxes, metals, or mixtures or composites thereof heated above their melting point. As another example, a suitable polymeric liquid may include a solution of one or more polymers in a suitable solvent, which solution forms a solid polymeric material upon removal of the solvent, for example, by evaporation. Such polymeric materials, which can be solidified from, for example, a melt state or by solvent evaporation, are well known to those of ordinary skill in the art. A variety of polymeric materials, many of which are elastomeric, are suitable, and are also suitable for forming molds or mold masters, for embodiments where one or both of the mold masters is composed of an elastomeric material. A non-limiting list of examples of such polymers includes polymers of the general classes of silicone polymers, epoxy polymers, and acrylate polymers. Epoxy polymers are characterized by the presence of a three-membered cyclic ether group commonly referred to as an epoxy group, 1,2-epoxide, or oxirane. For example, diglycidyl ethers of bisphenol A can be used, in addition to compounds based on aromatic amine, triazine, and cycloaliphatic backbones. Another example includes the well-known Novolac polymers. Non-limiting examples of silicone elastomers suitable for use according to the invention include those formed from precursors including the chlorosilanes such as methylchlorosilanes, ethylchlorosilanes, phenylchlorosilanes, etc.

Silicone polymers are used in certain embodiments, for example, the silicone elastomer polydimethylsiloxane. Non-limiting examples of PDMS polymers include those sold under the trademark Sylgard by Dow Chemical Co., Midland, MI, and particularly Sylgard 182, Sylgard 184, and Sylgard 186. Silicone polymers including PDMS have several beneficial properties simplifying fabrication of the microfluidic structures of the invention. For instance, such materials are inexpensive, readily available, and can be solidified from a prepolymeric liquid via curing with heat. For example, PDMSs are typically curable by exposure of the prepolymeric liquid to temperatures of about, for example, about 65 °C to about 75 °C for exposure times of, for example, about an hour. Also, silicone polymers, such as PDMS, can be elastomeric and thus may be useful for forming very small features with relatively high aspect ratios, necessary in certain embodiments of the invention. Flexible (e.g., elastomeric) molds or masters can be advantageous in this regard.

One advantage of forming structures such as microfluidic structures of the invention from silicone polymers, such as PDMS, is the ability of such polymers to be oxidized, for example by exposure to an oxygen-containing plasma such as an air plasma, so that the oxidized structures contain, at their surface, chemical groups capable of cross-linking to other oxidized silicone polymer surfaces or to the oxidized surfaces of a variety of other polymeric and non-polymeric materials. Thus, components can be fabricated and then oxidized and essentially irreversibly sealed to other silicone polymer surfaces, or to the surfaces of other substrates reactive with the oxidized silicone polymer surfaces, without the need for separate adhesives or other sealing means. In most cases, sealing can be completed simply by contacting an oxidized silicone surface to another surface without the need to apply auxiliary pressure to form the seal. That is, the pre-oxidized silicone surface acts as a contact adhesive against suitable mating surfaces. Specifically, in addition to being irreversibly sealable to itself, oxidized silicone such as oxidized PDMS can also be sealed irreversibly to a range of oxidized materials other than itself including, for example, glass, silicon, silicon oxide, quartz, silicon nitride, polyethylene, polystyrene, glassy carbon, and epoxy polymers, which have been oxidized in a similar fashion to the PDMS surface (for example, via exposure to an oxygen-containing plasma). Oxidation and sealing methods useful in the context of the present invention, as well as overall molding techniques, are described in the art, for example, in an article entitled "Rapid Prototyping of Microfluidic Systems and Polydimethylsiloxane," Anal. Chem., 70:474-480, 1998 (Duffy *et al*.), incorporated herein by reference.

In one set of embodiments, a subject having a condition such as a physiological condition to be analyzed (or other user, such as medical personnel) reads and/or otherwise determines a signal from a device. For example, the device may transmit a signal indicative of a condition of the subject and/or the device. Alternatively, or in addition, a signal produced by a device can be acquired in the form of a representation (e.g. a digitized signal, or the like) and transmitted to another entity for analysis and/or action. For example, a signal can be produced by a device, e.g., based on a sensor reading of an analyte, based on fluid delivered and/or received from the skin, based on a condition of the device, or the like. The signal may represent any suitable data or image. For example, the signal may represent the presence and/or concentration of an analyte in fluid received from a subject, the amount of fluid received from a subject and/or delivered to the subject, the number of times the device has been used, the battery life of the device, the amount of vacuum left in the device, the cleanliness or sterility of the device, the identity of the device (e.g., where multiple devices are given unique identification numbers, to prevent counterfeiting, accidental exchange of equipment to incorrect users, etc.), or the like. For instance, in one set of embodiments, an image of the signal (e.g., a visual image or photograph) can be obtained and transmitted to a different entity (for example, a user can take a cell phone picture of a signal generated by the device and send it, via cell phone, the other entity).

The other entity that the signal is transmitted to can be a human (e.g., a clinician) or a machine. In some cases, the other entity may be able to analyze the signal and take appropriate action. In one arrangement, the other entity is a machine or processor that analyzes the signal and optionally sends a signal back to the device to give direction as to activity (e.g., a cell phone can be used to transmit an image of a signal to a processor which, under one set of conditions, transmits a signal back to the same cell phone giving direction to the user, or takes other action). Other actions can include automatic stimulation of the device or a related device to dispense a medicament or pharmaceutical, or the like. The signal to direct dispensing of a pharmaceutical can take place via the same used to transmit the signal to the entity (e.g., cell phone) or a different vehicle or pathway. Telephone transmission lines, wireless networks, Internet communication, and the like can also facilitate communication of this type.

As one specific example, a device may be a glucose monitor. As signal may be generated by the device and an image of the signal captured by a cell phone camera and then transmitted via cell phone to a clinician. The clinician may then determine that the glucose (or e.g., insulin) level is appropriate or inappropriate and send a message indicating this back to the subject via cell phone.

Information regarding the analysis can also be transmitted to the same or a different entity, or a different location simply by removing the device or a portion of the device from the subject and transferring it to a different location. For example, a device can be used in connection with a subject to analyze presence and/or amount of a particular analyte. At some point after the onset of use, the device, or a portion of the device carrying a signal or signals indicative of the analysis or analyses, can be removed and, e.g., attached to a record associated with the subject. As a specific example, a patch or other device can be worn by a subject to determine presence and/or amount of one or more analytes qualitatively, quantitatively, and/or over time. The subject can visit a clinician who can remove the patch (or other device) or a portion of the patch and attach it to a medical record associated with the subj ect.

In some embodiments, the device may include a signal structure and a support structure. The support structure may be used, for example, for applying the substance transfer component or fluid transporter to the surface of the skin of the subject, e.g., so that fluid may be delivered and/or received from the skin of the subject. The signal structure may be used to indicate a state or condition of the device, e.g., of a condition of the device, and/or a condition of a fluid delivered or removed from the subject. For instance, the signal structure may indicate analysis of an analyte contained within a fluid removed from the subject. As discussed, the signal structure may be able to produce a signal visually (e.g., using a display, lights, etc.), by smell, sound, feel, taste, or the like.

In some cases, the signal structure may be integrally connected to the support structure. As used herein, the term "integrally connected," when referring to two or more objects, means objects that do not become separated from each other during the course of normal use, e.g., cannot be separated manually; separation requires at least the use of tools, and/or by causing damage to at least one of the components, for example, by breaking, peeling, etc. (separating components fastened together via adhesives, tools, etc.). For example, the device may be a one-use disposable item, or the device may be used multiple times.

In another set of embodiments, however, the signal structure may not be integrally connected to the support structure. Thus, the signal structure and the support structure may be separated from each other, in various embodiments. Separation may be performed, for example, by a user, or the separation may be automatically driven in some embodiments (e.g., a servo mechanism may cause one of the structures to become ejected or disconnected with the other, for example, similar to how a VCR ejects a tape).

In certain embodiments, the support structure and the signal structure are constructed and arranged to be connectable and/or detachable from each other readily by the subject. Thus, for instance, the subject (or another person) may be able to connect the support structure and the signal structure to assemble a device, and/or disconnect the support structure and the signal structure, without the use of tools such as screwdrivers or tape. In some cases, the connection and/or disconnection can occur while the device is affixed to the skin. Thus, for example, a device may be applied to the subject of the skin, and after use, one of the support structure and the signal structure may be removed from the skin of the subject, leaving the remainder of the device in place on the skin.

As an example, in one embodiment, a device may be fabricated to contain a first module that contains a support structure, and a second module containing the signal structure that is constructed and arranged for repeated connection and disconnection to the first module containing the support structure. The first module containing the support structure may be used to deliver and/or receive fluid from a subject. For instance, as discussed herein, the first module may contain a substance transfer component or a fluid transporter associated with the support structure for delivering and/or receiving fluid from the skin of the subject. The fluid may optionally be analyzed within the first module, and/or stored for later use, e.g., in a collection chamber. After receipt of sufficient fluid, the first module may be removed, leaving the second module in place, and optionally replaced with a new first module for subsequent use (e.g., for subsequent delivery and/or reception of fluid at a later time). In other embodiments, however, the second module may be removed, leaving the first module in place. Depending on the application, the removed module may be reused or disposed of (e.g., thrown in the trash), or the module may be shipped to another location for disposal and/or analysis, for example, to analyze fluid contained within the module, e.g., received from the subject. A module may be used once, or multiple times, before being removed from the device, depending on the application.

As another example, in some embodiments, a device may include a first portion for delivering and/or receiving fluid (or other substances) to or from the subject, and a second portion that is able to urge fluid to or from the subject, e.g., via the first portion. For example, in one set of embodiments, the first portion may comprise one or more substance transfer component or fluid transporters, and optionally a storage chamber for receiving a fluid received from the subject, a chamber for containing a fluid or other substance to be delivered to the subject (for example, an analgesic), an analytical chamber, or the like. For instance, in one set of embodiments, the first portion includes one or more needles or microneedles such as those described herein. The first portion may also contain, in certain cases, indicators, sensors, displays, microfluidic channels, activators, reversibly deformable structures, etc., as is discussed herein. The second portion may contain, for example, a vacuum chamber or other vacuum source which may be self-contained or pre-packaged, a pressure regulator such as a mechanical device (e.g., a pump, a syringe, a bulb, a Venturi tube, a plunger, a syringe pump, or the like).

In some cases, the first portion and the second portion can be separated from each other without breakage of one or both portions, or the use of tools. For example, the second portion may be used to cause fluid or other substances to be delivered and/or received to or from a subject, and optionally replaced between uses. For instance, after a second portion is activated to cause fluid delivery and/or reception, the second portion may be removed or ejected from the device and a fresh replacement added so that the device can be subsequently used. In some cases, the second portion may be removed and/or replaced without removing the device from the subject. Separation may be performed, for example, by a user, or the separation may be automatically driven in some embodiments (e.g., a servo mechanism as previously discussed).

In another aspect, the device may include an anticoagulant or a stabilizing agent for stabilizing the fluid received from the skin. The device may be a single, unitary device, or the device may contain one or more modules. For example, the fluid may be stored within the device for a certain period of time, and/or the device (or a portion thereof) may be shipped to another location for analysis or later use. For instance, a device may contain anticoagulant or a stabilizing agent in a storage chamber. As a specific non-limiting example, an anticoagulant may be used for blood received from the skin. Examples of anticoagulants include, but are not limited to, heparin, citrate, oxalate, or ethylenediaminetetraacetic acid (EDTA). Other agents may be used in conjunction or instead of anticoagulants, for example, stabilizing agents such as solvents, diluents, buffers, chelating agents, antioxidants, binding agents, preservatives, antimicrobials, or the like. Examples of preservatives include, for example, benzalkonium chloride, chlorobutanol, parabens, or thimerosal. Non-limiting examples of antioxidants include ascorbic acid, glutathione, lipoic acid, uric acid, carotenes, alpha-tocopherol, ubiquinol, or enzymes such as catalase, superoxide dismutase, or peroxidases. Examples of microbials include, but are not limited to, ethanol or isopropyl alcohol, azides, or the like. Examples of chelating agents include, but are not limited to, ethylene glycol tetraacetic acid or ethylenediaminetetraacetic acid. Examples of buffers include phosphate buffers such as those known to ordinary skill in the art.

In another aspect, the present invention is directed to a kit including one or more of the compositions previously discussed, e.g., a kit including a device for the delivery and/or receiving of fluid from the skin, a kit including a device able to create a pooled region of fluid within the skin of a subject, a kit including a device able to determine a fluid, or the like. A "kit," as used herein, typically defines a package or an assembly including one or more of the compositions or devices of the invention, and/or other compositions or devices associated with the invention, for example, as previously described. For example, in one set of embodiments, the kit may include a device and one or more compositions for use with the device. Each of the compositions of the kit, if present, may be provided in liquid form (e.g., in solution), or in solid form (e.g., a dried powder). In certain cases, some of the compositions may be constitutable or otherwise processable (e.g., to an active form), for example, by the addition of a suitable solvent or other species, which may or may not be provided with the kit. Examples of other compositions or components associated with the invention include, but are not limited to, solvents, surfactants, diluents, salts, buffers, emulsifiers, chelating agents, fillers, antioxidants, binding agents, bulking agents, preservatives, drying agents, antimicrobials, needles, syringes, packaging materials, tubes, bottles, flasks, beakers, dishes, frits, filters, rings, clamps, wraps, patches, containers, tapes, adhesives, and the like, for example, for using, administering, modifying, assembling, storing, packaging, preparing, mixing, diluting, and/or preserving the compositions components for a particular use, for example, to a sample and/or a subject.

A kit of the invention may, in some cases, include instructions in any form that are provided in connection with the compositions of the invention in such a manner that one of ordinary skill in the art would recognize that the instructions are to be associated with the compositions of the invention. For instance, the instructions may include instructions for the use, modification, mixing, diluting, preserving, administering, assembly, storage, packaging, and/or preparation of the compositions and/or other compositions associated with the kit. In some cases, the instructions may also include instructions for the delivery and/or administration of the compositions, for example, for a particular use, e.g., to a sample and/or a subject. The instructions may be provided in any form recognizable by one of ordinary skill in the art as a suitable vehicle for containing such instructions, for example, written or published, verbal, audible (e.g., telephonic), digital, optical, visual (e.g., videotape, DVD, etc.) or electronic communications (including Internet or web-based communications), provided in any manner.

In some embodiments, the present invention is directed to methods of promoting one or more embodiments of the invention as discussed herein. As used herein, "promoted" includes all methods of doing business including, but not limited to, methods of selling, advertising, assigning, licensing, contracting, instructing, educating, researching, importing, exporting, negotiating, financing, loaning, trading, vending, reselling, distributing, repairing, replacing, insuring, suing, patenting, or the like that are associated with the systems, devices, apparatuses, articles, methods, compositions, kits, etc. of the invention as discussed herein. Methods of promotion can be performed by any party including, but not limited to, personal parties, businesses (public or private), partnerships, corporations, trusts, contractual or sub-contractual agencies, educational institutions such as colleges and universities, research institutions, hospitals or other clinical institutions, governmental agencies, etc. Promotional activities may include communications of any form (e.g., written, oral, and/or electronic communications, such as, but not limited to, e-mail, telephonic, Internet, Web-based, etc.) that are clearly associated with the invention.

In one set of embodiments, the method of promotion may involve one or more instructions. As used herein, "instructions" can define a component of instructional utility (e.g., directions, guides, warnings, labels, notes, FAQs or "frequently asked questions," etc.), and typically involve written instructions on or associated with the invention and/or with the packaging of the invention. Instructions can also include instructional communications in any form (e.g., oral, electronic, audible, digital, optical, visual, etc.), provided in any manner such that a user will clearly recognize that the instructions are to be associated with the invention, e.g., as discussed herein.

The following documents are incorporated herein by reference: U.S. Patent Application Serial No. 12/478,756, filed June 4, 2009, entitled "Compositions and Methods for Rapid One-Step Diagnosis," by D. Levinson, published as U.S. Patent Application Publication No. 2010/0069726 on March 18, 2010; International Patent Application No. PCT/US09/046333, filed June 4, 2009, entitled "Compositions and Methods for Rapid One-Step Diagnosis," by D. Levinson, published as WO 2009/149308 on December 10, 2009; U.S. Patent Application Serial No. 12/716,233, filed March 2, 2010, entitled "Systems and Methods for Creating and Using Suction Blisters or Other Pooled Regions of Fluid within the Skin," by Levinson, et al.; U.S. Patent Application Serial No. 12/716,222, filed March 2, 2010, entitled "Oxygen Sensor," by Levinson, *et al*.; U.S. Patent Application Serial No. 12/716,229, filed March 2, 2010, entitled "Devices and Techniques Associated with Diagnostics, Therapies, and Other Applications, Including Skin-Associated Applications," by Bernstein, *et al*.; U.S. Patent Application Serial No. 12/716,226, filed March 2, 2010, entitled "Techniques and Devices Associated with Blood Sampling," by Levinson, *et al*.; U.S. Provisional Patent Application Serial No. 61/294,543, filed January 13, 2010, entitled "Blood Sampling Device and Method," by Chickering, *et al*.; U.S. Provisional Patent Application Serial No. 61/334,533, filed May 13, 2010, entitled "Rapid Delivery and/or Withdrawal of Fluids," by Chickering, *et al*.; and U.S. Provisional Patent Application Serial No. 61/334,529, filed May 13, 2010, entitled "Sampling Device Interfaces," by Chickering, *et al*.

Also incorporated herein by reference are U.S. Provisional Patent Application Serial No. 61/256,880, filed October 30, 2009, entitled "Systems and Methods for Altering or Masking Perception of Treatment of a Subject," by Chickering, *et al*.; U.S. Provisional Patent Application Serial No. 61/256,931, filed October 30, 2009, entitled "Modular Systems for Application to the Skin," by Bernstein, *et al*.; U.S. Provisional Patent Application Serial No. 61/294,543, filed January 13, 2010, entitled "Blood Sampling Device and Method," by Chickering, *et al*.; U.S. Provisional Patent Application Serial No. 61/334,533, filed May 13, 2010, entitled "Rapid Delivery and/or Withdrawal of Fluids," by Chickering, *et al*.; and U.S. Provisional Patent Application Serial No. 61/256,863, filed October 30, 2009, entitled "Systems and Methods for Treating or Shielding Blood on the Surface of the Skin," by Bernstein, *et al*. In addition, U.S. Provisional Patent Application Serial No. 61/373,764, filed August 13, 2010, entitled "Clinical and/or Consumer Techniques and Devices," by Chickering, *et al*. is hereby incorporated by reference in its entirety.

While several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present invention. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used. Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims and equivalents thereto, the invention may be practiced otherwise than as specifically described and claimed. The present invention is directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present invention.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

Embodiments of the invention will now be described in the following numbered paragraphs:
1. A method, comprising:
   providing a device for application to the skin of a subject, the device able to deliver and/or receive fluid to or from the subject;
   exposing the device to a scannable target; and
   applying the device to the skin of a subject.
2. The method of paragraph 1, wherein the scannable target is possessed by the subject.
3. The method of any one of paragraphs 1 or 2, wherein the scannable target is attached to the subject.
4. The method of any one of paragraphs 1-3, wherein the scannable target comprises an RFID tag.
5. The method of any one of paragraphs 1-4, wherein the scannable target comprises a printed pattern.
6. The method of any one of paragraphs 1-5, wherein the scannable target comprises a bar code.
7. The method of any one of paragraphs 1-6, wherein the scannable target comprises a 1-dimensional bar code.
8. The method of any one of paragraphs 1-7, wherein the scannable target comprises a 2-dimensional bar code.
9. The method of any one of paragraphs 1-8, wherein the device comprises a substance transfer component for delivering and/or receiving fluid to or from the subject.
10. The method of paragraph 9, wherein the substance transfer component comprises one or more microneedles.
11. A device for receiving a substance from the skin and/or from beneath the skin of a subject, and/or for delivering a substance to the skin and/or to a location beneath the skin of a subject, the device comprising:
   a substance transfer component;
   a scanner; and
   an activator constructed and arranged to insert the substance transfer component into the skin of the subject after receiving a signal from the scanner.
12. The device of paragraph 11, wherein the scanner is able to detect radio waves.
13. The device of paragraph 11, wherein the scanner is an optical scanner.
14. The device of paragraph 11, wherein the scanner is able to detect sound waves.
15. The device of any one of paragraphs 11-14, wherein the substance transfer component comprises one or more microneedles.
16. A device for receiving a substance from the skin and/or from beneath the skin of a subject, and/or for delivering a substance to the skin and/or to a location beneath the skin of a subject, the device comprising:
   a plurality of microneedles mounted on a rotatable structure; and
   an activator constructed and arranged to insert one or more of the microneedles into the skin of the subject.
17. The device of paragraph 16, wherein the rotatable structure can be removed from the device without tools or breakage.
18. A device for receiving a substance from the skin and/or from beneath the skin of a subject, and/or for delivering a substance to the skin and/or to a location beneath the skin of a subject, the device comprising:
   one or more microneedles; and
   a sleeve into which the microneedles can be withdrawn when the microneedles are not being used to deliver and/or receive fluid from the skin of a subject.
19. The device of paragraph 18, wherein the sleeve can be moved by the device to expose the microneedles.
20. The device of any one of paragraphs 18 or 19, wherein the microneedles can be extended and/or retracted into the device.
21. The device of any one of paragraphs 18-20, wherein the sleeve is integrally formed within the device.
22. A device for receiving a substance from the skin and/or from beneath the skin of a subject, the device comprising:
   a substance transfer component;
   a storage chamber for receiving a fluid received from the subject, the storage chamber in fluid communication with the substance transfer component; and
   a reservoir able to contain a fluid deliverable to the storage chamber.
23. The device of paragraph 22, wherein the substance transfer component comprises one or more microneedles.
24. The device of any one of paragraphs 22 or 23, wherein the fluid is a diluent.
25. The device of any one of paragraphs 22-24, wherein the fluid contains a buffer.
26. A device for receiving a substance from the skin and/or from beneath the skin of a subject, the device comprising:
   a substance transfer component; and
   a storage chamber for receiving a fluid received from the subject, the storage chamber in fluid communication with the substance transfer component, wherein the storage chamber contains a fluid.
27. The device of paragraph 26, wherein the fluid is a diluent.
28. The device of any one of paragraphs 26 or 27, wherein the fluid contains a buffer.
29. The device of any one of paragraphs 26-28, wherein the substance transfer component comprises one or more microneedles.

It should be understood that various changes and modifications to the presently preferred embodiments described herein will be apparent to those skilled in the art. Such changes and modifications can be made without departing from the spirit and scope of the present invention and without diminishing its intended advantages. It is therefore intended that such changes and modifications be covered by the following claims.

## Claims

1. A device for receiving a substance from the skin and/or from beneath the skin of a subject, the device comprising:
a substance transfer component;
a storage chamber for receiving a fluid received from the subject, the storage chamber in fluid communication with the substance transfer component; and
a reservoir able to contain a fluid deliverable to the storage chamber.

2. A device for receiving a substance from the skin and/or from beneath the skin of a subject, the device comprising:
a substance transfer component; and
a storage chamber for receiving a fluid received from the subject, the storage chamber in fluid communication with the substance transfer component, wherein the storage chamber contains a fluid.

3. The device of any one of claims 1 or 2, wherein the fluid is a diluent or contains a buffer.

4. The device of any preceding claim, wherein the substance transfer component comprises a plurality of microneedles.

5. A device for receiving a substance from the skin and/or from beneath the skin of a subject, and/or for delivering a substance to the skin and/or to a location beneath the skin of a subject, the device comprising:
a plurality of microneedles mounted on a rotatable structure; and
an activator constructed and arranged to insert one or more of the microneedles into the skin of the subject.

6. The device of claim 5, wherein the rotatable structure can be removed from the device without tools or breakage.

7. The device of any of claims 4-6, further comprising a deployment actuator comprising a reversibly deformable structure able to drive the plurality of microneedles into the skin of the subject.

8. The device of claim 7, wherein the reversibly deformable structure has a first stable configuration and a second stable configuration, the reversibly deformable structure switchable between the first stable configuration and the second stable configuration.

9. The device of any of claims 7-8, wherein the deployment actuator is able to insert the plurality of microneedles into the skin of the subject at a speed of at least about 1 cm/s or an acceleration of at least about 100,000 m/s² at the point of initial contact with the skin.

10. The device of any of claims 7-9, wherein the reversibly deformable structure comprises a flexible concave member or a snap dome.

11. The device of any of claims 7-10, wherein the device further comprises a triggering mechanism that initiates deployment of the plurality of microneedles into the skin of the subject.

12. The device of any of claims 7-11, wherein the device further comprises a pre-packaged vacuum chamber having an internal pressure less than atmospheric pressure prior to the point at which the device is used on the subject.
